(19) Europäisches Patentamt
European Patent Office
Office européen des brevets

(11) Veröffentlichungsnummer: **0 358 025 B1**

(12) # EUROPÄISCHE PATENTSCHRIFT

(45) Veröffentlichungstag der Patentschrift: **20.07.94**

(51) Int. Cl.$^{5}$: **C07D 235/24**, C08K 5/3447, C07D 235/20

(21) Anmeldenummer: **89115356.1**

(22) Anmeldetag: **19.08.89**

Die Akte enthält technische Angaben, die nach dem Eingang der Anmeldung eingereicht wurden und die nicht in dieser Patentschrift enthalten sind.

(54) **N-substituierte Benzimidazol-2-carbonsäureanilide, deren Verwendung als Lichtschutzmittel, insbesondere Polymere und diese Anilide enthaltendes organisches Material.**

(30) Priorität: **23.08.88 DE 3828537**

(43) Veröffentlichungstag der Anmeldung: **14.03.90 Patentblatt 90/11**

(45) Bekanntmachung des Hinweises auf die Patenterteilung: **20.07.94 Patentblatt 94/29**

(84) Benannte Vertragsstaaten: **BE CH DE ES FR GB IT LI NL**

(56) Entgegenhaltungen:
**EP-A- 0 284 828**
**EP-A- 4 011 236**
**US-A- 3 907 700**

(73) Patentinhaber: **BASF Aktiengesellschaft**
**Carl-Bosch-Strasse 38**
**D-67063 Ludwigshafen(DE)**

(72) Erfinder: **Spang, Peter, Dr.**
**Zur Audell 43**
**D-6670 St. Ingbert(DE)**
Erfinder: **Neumann, Peter, Dr.**
**Poststrasse 2**
**D-6800 Mannheim 31(DE)**
Erfinder: **Wagenblast, Gerhard, Dr.**
**Hanns-Fay-Strasse 3**
**D-6710 Frankenthal(DE)**
Erfinder: **Trauth, Hubert**
**Milanstrasse 5**
**D-6724 Dudenhofen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Beschreibung**

Aus den US-A 3 907 700 und 4 011 236 sind bereits Benzimidazolderivate als UV-Absorber für Kunststoffe bekannt. Die dort beschriebenen N-(Benzimidazol-2-yl)-phenylcarboxamide der Formel (X)

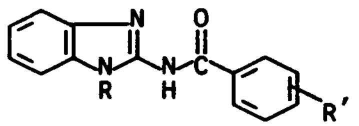

(X)

besitzen zwar im UV-Bereich von 280 bis 350 nm starke Absorptionsbanden, genügen jedoch in ihren anwendungstechnischen Eigenschaften nicht dem heutigen Stand der Technik.

Von Nachteil sind bei diesen Verbindungen die schlechte Löslichkeit, ihre Neigung zur Migration und ihre zu geringe Lichtschutzwirkung insbesondere in strahlungsempfindlichen Kunststoffen, wie Polyurethanen.

Aufgabe der Erfindung war es, Lichtschutzmittel für organische Materialien, insbesondere Polymere, zu entwickeln, welche die Nachteile der Schutzmittel des Standes der Technik nicht aufweisen.

Gegenstand der vorliegenden Erfindung sind Verbindungen der Formel (I)

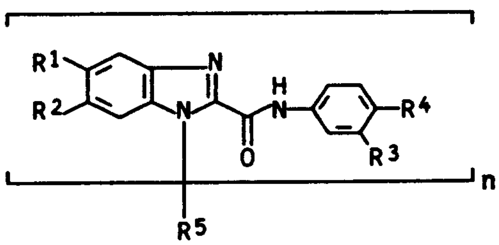

(I),

in der

| | |
|---|---|
| $R^1$ und $R^2$ | unabhängig voneinander für H, Cl, $C_1$- bis $C_4$-Alkyl, $C_1$- bis $C_4$-Alkoxy, gegebenenfalls durch $C_1$- bis $C_4$-Alkyl oder $C_1$- bis $C_4$-Alkoxy substituiertes Phenyl oder $C_7$- bis $C_9$-Phenylalkyl, |
| $R^3$ und $R^4$ | unabhängig voneinander für H, $C_1$- bis $C_{18}$-Alkyl, durch -O-ein- oder mehrfach unterbrochener $C_3$- bis $C_{18}$-Alkyl, $C_1$- bis $C_{18}$-Alkoxy, durch -O- ein- oder mehrfach unterbrochenes $C_4$- bis $C_{18}$-Alkoxy, gegebenenfalls durch $C_1$- bis $C_4$-Alkyl oder $C_1$- bis $C_4$-Alkoxy substituiertes Phenyl, $C_7$- bis $C_{15}$-Phenylalkyl, Phenoxy, $C_7$- bis $C_{15}$-Phenylalkyloxy, $C_1$- bis $C_{12}$-Alkylcarbonylamino, Benzoylamino, $C_1$-bis $C_{12}$-Alkanoyloxy oder Benzoyloxy, oder |
| $R^3$ und $R^4$ | zusammen für Methylendioxy oder Ethylendioxy, |
| n | für 1 oder 2 und |
| $R^5$ | a) - wenn n=1 ist - für $C_5$- bis $C_{18}$-Alkyl, $C_2$- bis $C_{10}$-Hydroxyalkyl, $C_5$- bis $C_{12}$-Cycloalkyl, $C_2$- bis $C_{18}$-Alkenyl, wobei Cycloalkyl und Alkenyl durch OH substituiert sein können, durch -O- ein- oder mehrfach unterbrochenes $C_3$-bis $C_{18}$-Alkyl, durch $C_1$- bis $C_4$-Alkyl oder $C_1$- bis $C_4$-Alkoxy substituiertes $C_8$- bis $C_{15}$-Phenylalkyl oder für einen Rest der Formeln |

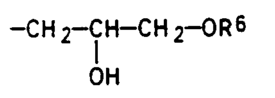 ,

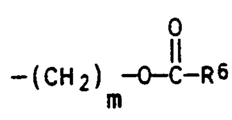 ,

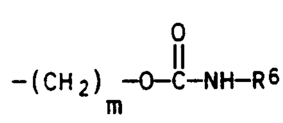 oder 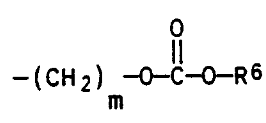

worin

m 2 bis 4 und

$R^6$ $C_1$- bis $C_{18}$-Alkyl, $C_2$- bis $C_{18}$-Alkenyl, $C_5$- bis $C_{12}$-Cycloalkyl, $C_7$-bis $C_{15}$-Phenylalkyl, gegebenenfalls durch $C_1$- bis $C_4$-Alkyl, $C_1$- bis $C_4$-Alkoxy oder C1 substituiertes Phenyl oder Naphthyl bedeuten, oder b) - wenn n = 2 ist - für $C_2$- bis $C_{10}$-Alkylen, $C_5$- bis $C_{12}$-Cycloalkylen, wobei Alkylen und Cycloalkylen durch -OH substituiert sein können, $C_4$- bis $C_{12}$-Alkenylen, durch -O- ein oder mehrfach unterbrochenes $C_4$- bis $C_8$-Alkylen oder für einen Rest der Formeln

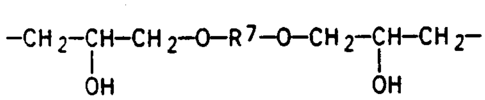 , 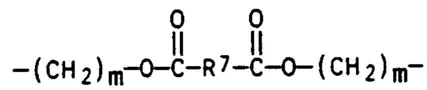 ,

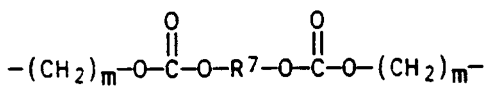 oder 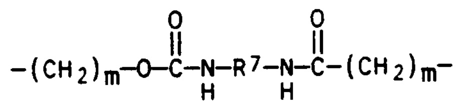

stehen, worin

$R^7$ $C_2$- bis $C_8$-Alkylen, $C_2$- bis $C_8$-Alkenylen, $C_5$- bis $C_{12}$-Cycloalkylen, gegebenenfalls durch $C_1$- bis $C_4$-Alkyl oder $C_1$- bis $C_4$-Alkoxy substituiertes Phenylen, Naphthylen, Diphenylen oder einen Rest der Formeln

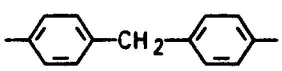 , 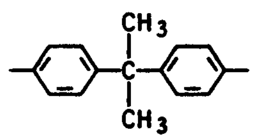 , 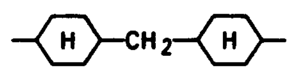 oder

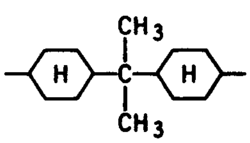

bedeutet.

Die Verbindungen (I) der Erfindung sind wirksame Lichtstabilisatoren für organische Materialien, z.B. für eine große Anzahl von Polymeren.

Die erfindungsgemäßen Verbindungen sind nicht nur wirksame Lichtschutzmittel, sondern aufgrund ihrer geringen Flüchtigkeit bei höheren Temperaturen besonders geeignet zum Stabilisieren von Polymeren, welche bei höheren Temperaturen verarbeitet werden müssen.

$R^1$ und $R^2$ stehen vorzugsweise für Wasserstoff, Chlor, $C_1$- bis $C_4$-Alkyl wie Methyl, Ethyl, n- und i-Propyl, n-, i- und t-Butyl, $C_1$- bis $C_4$-Alkoxy wie Methoxy, Ethoxy, Propoxy und Butoxy, für Phenyl, Benzyl, $\alpha$-Methylbenzyl, $\alpha,\alpha$-Dimethylbenzyl.

Für $R^1$ und $R^2$ ist Wasserstoff besonders bevorzugt.

Für

$R^3$ und $R^4$ sind bevorzugt Wasserstoff, $C_1$- bis $C_{12}$-Alkyl, durch -O-einfach oder mehrfach unterbrochenes $C_3$- bis $C_{10}$-Alkyl, $C_1$- bis $C_{12}$-Alkoxy, durch -O- ein oder mehrfach unterbrochenes $C_4$- bis $C_{12}$-Alkoxy, $C_7$- bis $C_9$-Phenylalkyl, Phenoxy, $C_7$- bis $C_9$-Phenylalkoxy, $C_1$- bis $C_{12}$-Alkylcarbonylamino, Benzoylamino, $C_1$- bis $C_{12}$-Alkanoyloxy oder Benzoyloxy.

$R^3$ und $R^4$ können zusammen auch Methylendioxy oder Ethylendioxy sein.

Neben den bestimmt genannten Substituenten $R^3$ und $R^4$ sind im einzelnen z.B. zu nennen:

$\alpha$) $C_1$-$C_{12}$-Alkyl: Methyl, Ethyl, n- und i-Propyl, n-Butyl, 2-Butyl, tert.-Butyl, n-Pentyl, tert.-Pentyl, n-Hexyl, n-Heptyl, n-Octyl, 2-Ethylhexyl, n-Nonyl, n-Decyl und n-Dodecyl;

$\beta$) $C_1$-$C_{12}$-Alkoxy: Methoxy, Ethoxy, i-Propoxy, n-Butoxy, n-Pentoxy, i-Pentoxy, n-Hexoxy, n-Heptoxy, n-Octoxy, 2-Ethylhexoxy, n-Nonoxy, n-Decoxy und n-Dodecoxy;

$\gamma$) Benzyloxy, 2-Phenylethoxy und 3-Phenylpropoxy.

Besonders bevorzugt sind Verbindungen (I), bei denen

$R^3$ für H und

$R^4$ für $C_1$- bis $C_{12}$-Alkyl, $C_1$- bis $C_{12}$-Alkoxy, Phenoxy, $C_1$- bis $C_{12}$-Alkylcarbonylamino oder $C_1$- bis $C_{12}$-Alkanoyloxy stehen.

Die Bedeutung von $R^5$ ist davon abhängig, ob n = 1 oder 2 ist.

Wenn n = 1 ist, dann steht

$R^5$ für $C_5$- bis $C_{18}$-Alkyl, $C_2$- bis $C_{10}$-Hydroxyalkyl, $C_5$- bis $C_{12}$-Cycloalkyl, $C_2$- bis $C_{18}$-Alkenyl, durch -O- ein oder mehrfach unterbrochenes $C_3$- bis $C_{18}$-Alkyl, durch $C_1$- bis $C_4$-Alkyl oder $C_1$- bis $C_4$-Alkoxy substituiertes $C_8$- bis $C_{15}$-Phenylalkyl oder für einen Rest der Formeln

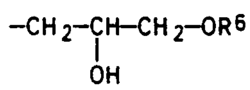 , 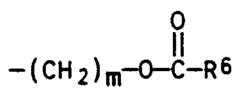 ,

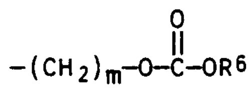 oder 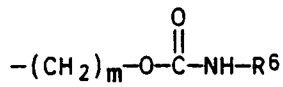 ,

worin

m 2, 3 oder 4 und

$R^6$ $C_1$- bis $C_{18}$-Alkyl, $C_2$- bis $C_{18}$-Alkenyl, $C_5$- bis $C_{12}$-Cycloalkyl, $C_7$- bis $C_9$-Phenylalkyl, gegebenenfalls durch $C_1$- bis $C_4$-Alkyl oder $C_1$- bis $C_4$-Alkoxy substituiertes Phenyl oder Naphthyl bedeuten.

Bevorzugt steht in diesem Fall $R^5$ für $C_5$- bis $C_{12}$-Alkyl, $C_5$-$C_{12}$-Cycloalkyl, für $C_2$-$C_4$-Hydroxyalkyl wie Hydroxyethyl, Hydroxypropyl, Hydroxyisopropyl, Hydroxybutyl, $CH_2CH_2OCH_2CH_3$, $CH_2CH_2OC_4H_9$, $CH_2CH_2OCH_2CH_2OCH_3$, $CH_2CH_2OCH_2CH_2OC_2H_5$, $CH_2CH_2OCH_2CH_2OC_4H_9$,

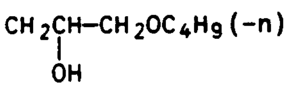 , 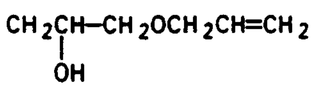 oder 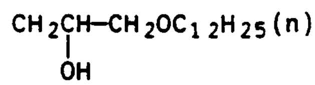

Vorzugsweise ist

m 2 und

$R^6$ $C_1$- bis $C_{18}$-Alkyl, $C_5$- bis $C_{12}$-Cycloalkyl, $C_1$- bis $C_{18}$-Alkenyl, Benzyl, Phenylethyl, Phenylpropyl, Phenyl, durch $C_1$- bis $C_4$-Alkyl oder $C_1$- bis $C_4$-Alkoxy substituiertes Phenyl oder Naphthyl.

Im Fall von n = 2 steht $R^5$ vorzugsweise für $C_2$- bis $C_{10}$-Alkylen, $C_4$- bis $C_{12}$-Alkenylen, durch -O- ein- oder mehrfach unterbrochenes $C_4$- bis $C_8$-Alkylen oder für einen zweiwertigen Rest der Formeln

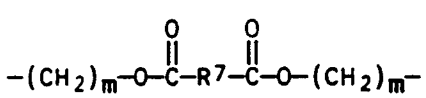 oder 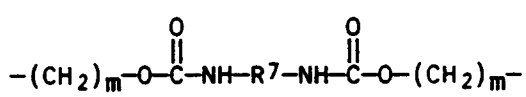

worin m 2, 3 oder 4, insbesondere 2 ist. $R^7$ steht vorzugsweise für $C_2$-$C_8$-Alkylen.

Die Verbindungen der Formel (I) können nach an sich bekannten Verfahren hergestellt werden.

Vorzugsweise geht man bei der Herstellung der Verbindungen mit der Formel (I) von Benzimidazol-2-carbonsäureanilidderivaten der Formel (II) aus, die in der EP-A-0 284 828 beschrieben sind.

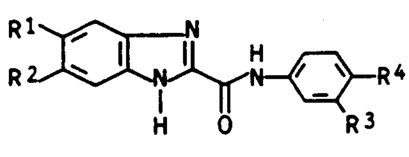 (II)

So sind die Verbindungen (I) durch Umsetzen von Aniliden der Formel (II) mit Verbindungen der Formel (III)

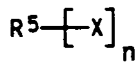 (III),

in der

X eine Abgangsgruppe, wie Cl, Br oder

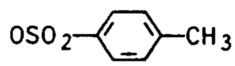

darstellt, gut zugänglich.

X steht vorzugsweise für Cl oder Br. Diese Umsetzungen werden unter den für N-Alkylierungen üblichen Bedingungen durchgeführt. Als Lösungsmittel kommen beispielsweise Dimethylformamid, Dimethylacetamid, N-Methylpyrolidon, Ethylenglykoldiether und aliphatische Ketone, wie Aceton, Cyclohexanon in Betracht.

Bevorzugt sind Dimethylformamid, N-Methylpyrrolidon und Cyclohexanon als Reaktionsmedien.

Als Hilfsbasen kommen für diese Umsetzungen die üblichen, z.B. KOH, NaOH, $K_2CO_3$, $Na_2CO_3$, $KHCO_3$, $NaHCO_3$ oder auch Natriummethylat oder Natriumethanolat in Betracht. Besonders bevorzugt sind dabei je nach Art des Restes $R^5$ und der Abgangsgruppe X KOH, $K_2CO_3$ oder $KHCO_3$.

Die Reaktionstemperaturen liegen zwischen Raumtemperatur und 160 °C, vorzugsweise zwischen 60 °C und 140 °C, insbesondere zwischen 80 und 110 °C.

Verbindungen der Formel (I), in der

$R^5$ für den Rest $-CH_2-CH(OH)-CH_2-O-R^6$ steht, können durch Reaktion von Aniliden der Formel (II) mit Glydcidylethern der Formel (IVa) hergestellt werden.

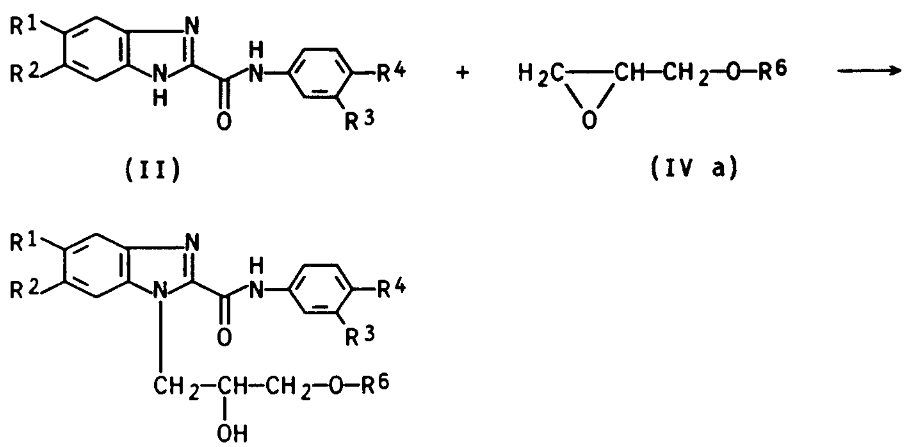

(II) (IV a)

Durch Umsetzung von zwei Äquivalenten der Anilide (II) mit 1 Mol Diglycidylether der Formel (IVb)

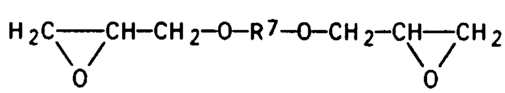 (IV b)

sind analog Verbindungen (I), in denen n = 2 und

$R^5$ $-CH_2-CH(OH)-CH_2-O-R^7-O-CH_2-CH(OH)-CH_2-$ sind, herstellbar.

Die Umsetzung mit (IVa) oder (IVb) erfolgt in inerten Lösungsmitteln, wie z.B. Toluol, Dimethylformamid in Gegenwart einer Base, wie Piperidin, Triethylamin, $K_2CO_3$ oder $Na_2CO_3$ bei Temperaturen zwischen 50 und 120 °C.

Verbindungen der Formel (I), in der

$R^5$ für einen der Reste

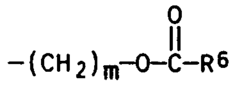 , 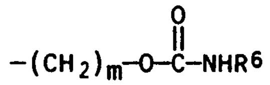 , 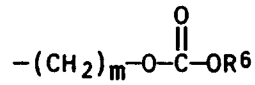 bzw.

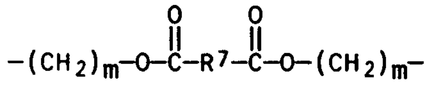 , 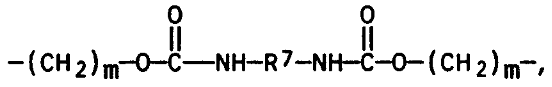

oder 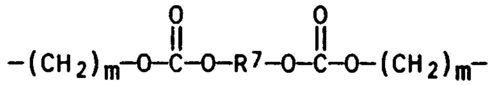

steht, können durch Umsetzen von Aniliden der Formel (V)

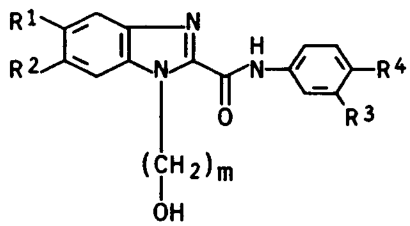 (V)

entweder mit

a) Mono- und Dicarbonsäurederivaten der Formeln (VIa) bzw. (VIb)

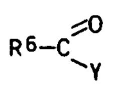 (VI a) , 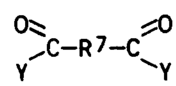 (VI b)

in denen

Y für Cl, $OCH_3$, $OC_2H_5$ oder $OC_6H_5$ steht, oder mit

b) Mono- bzw. Diisocyanaten der Formeln (VIIa) bzw. (VIIb)

$O = C = N\text{-}R^6$ (VII a), $O = C = N\text{-}R^7\text{-}N = C = O$ (VII b)

oder mit

c) Mono- bzw. Dichlorameisensäureestern der Formel (VIIIa) bzw. (VIIIb)

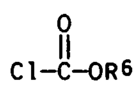 (VIII a) , 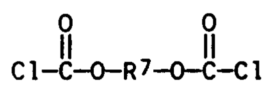 (VIII b)

synthetisiert werden.

Die Verbindungen (I) werden nach üblichen Verfahren in die Polymeren eingearbeitet. Die Einarbeitung kann beispielsweise durch Einmischen der Verbindungen und gegebenenfalls weiterer Additive in die Schmelze nach der in der Technik üblichen Methode vor oder während der Formgebung, oder auch durch Aufbringen der gelösten oder dispergierten Verbindungen auf das Polymer direkt oder durch Einmischen in eine Lösung, Suspension oder Emulsion des Polymeren, gegebenenfalls unter nachträglichem Verdunstenlassen des Lösungsmittels, erfolgen.

Als Polymere kommen z.B. in Betracht: Polyolefine, Polystyrol, Styrolpolymerisate, halogenhaltige Vinylpolymere, Polyacrylate, Polymethacrylate, Polyacrylamide, Polyacrylnitril, Polyvinylalkohol und dessen Acylderivate, Polyacetate, Polyalkylenoxide, Polyphenylenoxide, Polyurethane und Polyharnstoffe, Polysulfone, Polyamide, Polyester, Polycarbonate, vernetzte Polymere aus Aldehyden und Phenolen, Harnstoff oder Melamin, ungesättigte Polyesterharze, Alkydharze, duroplastische und thermoplatische Acrylharze.

Gegenstand der Erfindung sind außerdem stabilisierte organische Materialien, insbesondere synthetische Polymere, welche, bezogen auf das zu stabilisierende Material 0,05 bis 10, vorzugsweise 0,1 bis 5 Gew.-% einer oder mehrer Verbindungen der Formel I enthalten.

Wegen ihrer sehr geringen Flüchtigkeit sind die erfindungsgemäßen Verbindungen vorzugsweise zum Stabilisieren von Polyestern wie z.B. Polyäthylenterephthalat, Polybutylenterephthalat oder deren Copolymeren, von Polycarbonaten, z. B. aus Bisphenol A und Phosgen oder deren Copolymeren, von Polyamiden, z. B. Nylon-6, Nylon-6,6, Nylon-6,10 etc. sowie Copolymere, von MF- und UF-Harzen, von heißvernetzbaren und thermoplastischen Acrylharzen und von Polyurethanen hervorragend geeignet.

Bevorzugt werden Lacke aus derartigen Polymeren stabilisert.

Die so stabiliserten Materialien können in die für die Anwendung üblichen Formen überführt werden, z.B. Folien, Fasern, Bändchen, Profile oder zur Herstellung von Bindemitteln für Lacke, Klebstoffe, Kitte oder Formmassen verwendet werden.

In der Praxis können die Verbindungen der Formel I zusammen mit 0,1 bis 5, vorzugsweise 0,5 bis 3 Gew.-% weiteren üblichen Zusatzstoffen, wie Antioxidantien, weitere Lichtstabilisatoren, oder Gemische davon angewendet werden.

Solche üblichen Zusatzstoffe sind z.B.: Antioxidantien, UV-Absorber und Lichtschutzmittel wie 2-(2'-Hydroxyphenyl)-benztriazole, 2,4-Bis-(2'-hydroxyphenyl)-6-alkyl-s-triazine, 2-Hydroxybenzophenone, 1,3-Bis-(2'-hydroxybenzoyl)-benzole, Ester von gegebenenfalls substituierten Benzoesäuren, Acrylate, außerdem Nickelverbindungen, sterisch gehinderte Amine, Metalldesaktivatoren, Phosphite, peroxidzerstörende Verbindungen, Polyamidstabilisatoren, basische Co-Stabilisatoren, Nukleierungsmittel und sonstige Zusätze wie Weichmacher, Gleitmittel, Emulgatoren, Füllstoffe, Ruß, Kaolin, Talk, Glasfasern, Pigmente, optische Aufheller, Flammschutzmittel und Antistatica.

Die Verbindungen der Formel (I) eignen sich hervorragend zum Schutz von ABS-Polymeren und insbesondere von Polyurethanen, die sich von Polyethern, Polyestern und Polybutadien mit endständigen Hydroxylgruppen und aliphatischen oder aromatischen Polyisocyanaten ableiten, sowie deren Vorprodukte gegen Abbau durch Wärme und insbesondere durch Einwirkung von Licht.

Die stabilisierende Wirkung kann noch gesteigert werden, wenn man zusätzlich bekannte Antioxidantien, z.B. Verbindungen auf der Basis sterisch gehinderter Phenole oder Schwefel oder Phosphor enthaltende Costabilisatoren mitverwendet.

Als derartige phenolische Antioxidationsmittel kommen z. B. 2,6-Di-tert.-butyl-4-methylphenol, n-Octadecyl-$\beta$-(3,5-di-tert.-butyl-4-hydroxyphenyl)-propionat, 1,1,3-Tris-(2-methyl-4-hydroxy-5-tert.-butyl-phenyl)-butan, 1,3,5-Trimethyl-2,4,6-tris-(3,5-di-tert.-butyl-4-hydroxy-benzyl)-benzol, 1,3,5-Tris-(3,5-di-tert.-butyl-4-hydroxybenzyl)-isocyanurat, 1,3,5-Tris-[3,5-di-tert.-butyl-4-hydroxyphenyl)-propionyloxy-ethyl]-isocyanurat, 1,3,5-Tris-(2,6-di-methyl-3-hydroxy-4-tert.-butylbenzyl)-isocyanurat, Pentaerythrit-tetrakis-[$\beta$-(3,5-di-tert.-butyl-4-hydroxy-phenyl)-propionat] in Betracht.

Als phosphorhaltige Antioxidantien sind z. B. zu nennen: Tris-(nonylphenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit, Tetrakis-(2,4-di-tert.-butylphenyl)-4,4'-biphenylendiphosphit.

Als Schwefel enthaltende Antioxidationsmittel sind z.B. Dilaurylthiodipropionat, Dimyristylthiodipropionat, Distearylthiodipropionat, Pentaerythrittetrakis-($\beta$-laurylthiopropionat), Pentaaerythrittetrakis-($\beta$-hexylthiopropionat) zu nennen.

Eine besonders gute Stabilisierung erzielt man, wenn man zu den Verbindungen der Formeln (I), noch mindestens einen Lichtstabilisator aus der Verbindungsklasse der sterisch gehinderten Amine in üblicher Konzentration zusetzt.

Als sterisch gehinderte Amine kommen z. B. in Betracht: Bis-(2,2,6,6-tetra-methylpiperidyl)-sebacat, Bis-(1,2,2,6,6-pentamethyliperidyl)-sebacat, Bis-(1,2,2,6,6-pentamethylpiperidyl)-ester, das Kondensationsprodukt von 1-Hydroxyethyl-2,2,6,6-tetramethyl-4-hydroxypiperidin und Bernsteinsäure, das Kondensationsprodukt von N,N'-(2,2,6,6-Tetramethylpiperidyl)-hexymethylendiamin und 4-tert.-Octylamino-2,6-dichlor-1,3,5-s-triazin, Tris-(2,2,6,6-Tetramethylpiperidyl)-nitrilotriacetat, Tetrakis-(2,2,6,6-tetramethyl-4-piperidyl)-1,2,3,4-butan-tetracarbonsäure, 1,1'-(1,2-Ethandiyl)-bis-(3,3,5,5-tetramethylpiperazinon), die Kondensationsprodukte von 4-Amino-2,2,6,6-tetramethylpiperidinen und Tetramethylolacetylendiharnstoffen.

Eine ganz besonders gute Stabilisierung von Polyurethanen erhält man, wenn das Polyurethan mit einem Gemisch aus mindestens einer Verbindung der Formel (I), mindestens einem der oben genannten Antioxidantien und mindestens einer der sterisch gehinderten Aminverbindungen stabilisiert.

Die folgenden Beispiele sollen die Erfindung zusätzlich erläutern.

A) Herstellungsbeispiele

Beispiel 1

14,1 g (0,05 mol) 4'-Ethoxy-benzimidazol-2-carbonsäureanilid der Formel

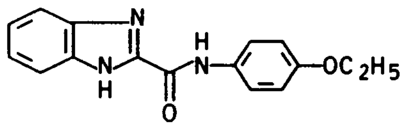

und 8,3 g (0,06 mol) $K_2CO_3$ wurden zusammen mit 9,9 g (0,06 mol) 1-Bromhexan in 30 ml Dimethylformamid suspendiert und 3 Stunden bei 85 bis 90 °C gerührt.

Anschließend wurde die heiße Reaktionsmischung filtriert und das Filtrat im Vakuum eingeengt. Der ölige Rückstand wurde aus ca. 120 ml Methanol umkristallisiert. Ausbeute: 14,0 g farbloses Produkt der Formel

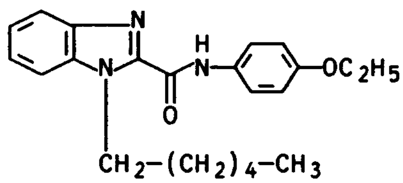

Schmp.: 72 - 73 °C.

| Analyse: $C_{22}H_{27}N_3O_2$ (365,5) | | | | | | | | |
|---|---|---|---|---|---|---|---|---|
| Ber.: | C | 72,3 | H | 7,44 | N | 11,49 | O | 8,76 |
| Gef.: | C | 72,4 | H | 7,6 | N | 11,1 | O | 8,9 |

UV: $\lambda_{max}$ [nm] ($\epsilon \cdot 10^{-3}$) = 306 (20,5) in $CH_3OH$

Beispiel 2

14,1 g (0,05 mol) 4'-Ethoxy-benzimidazol-2-carbonsäureanilid, 7,0 g (0,07 mol) $KHCO_3$ und 13,5 g (0,07 mol) 1-Bromoctan wurden in 25 ml Dimethylformamid 6,5 Stunden auf 100 bis 105 °C erhitzt. Die Reaktionslösung wurde dann heiß von dem ausgefallenen NaBr abfiltriert. Nach dem Abziehen des Lösungsmittels wurde der Rückstand aus Methanol (ca. 100 ml) umkristallisiert. Ausbeute: 14,8 g farblose Kristalle des Produktes der Formel

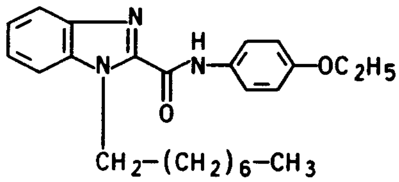

Schmp.: 67 - 68 °C.

| Analyse: $C_{24}H_{31}N_3O_2$ (393,6) | | | | | | | | |
|---|---|---|---|---|---|---|---|---|
| Ber.: | C | 73,28 | H | 7,90 | N | 10,68 | O | 8,14 |
| Gef.: | C | 73,2 | H | 7,8 | N | 10,4 | O | 8,2 |

UV: $\lambda_{max}$ [nm] ($\epsilon \cdot 10^{-3}$) = 305 (20,2)

Beispiele 3 bis 10

Analog Beispiel 1 oder 2 wurden unter Verwendung der entsprechend substituierten Benzimidazol-2-carbonsäureanilide der Formel (II) und der entsprechenden Brom- oder Chlorverbindungen der Formel (III) die in Tabelle 1 angegebenen Verbindungen (I) hergestellt.

Tabelle 1

| Beispiel | Verbindung (I) | Schmp. [°C] | Analyse | C | H | N | O | UV ($CH_3OH$) $\lambda_{max}$ [nm] ($\varepsilon \cdot 10^{-3}$) |
|---|---|---|---|---|---|---|---|---|
| 3 | 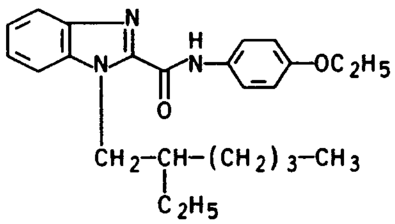 | 80 - 82 | b. | 73,28 | 7,90 | 10,68 | 8,14 | 305 |
| | | | g. | 73,2 | 8,1 | 10,6 | 8,1 | (21,5) |
| 4 | 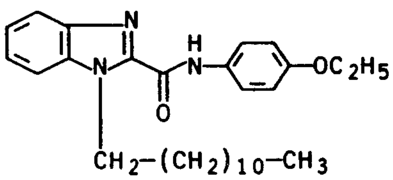 | 70 - 71 | b. | 74,83 | 8,69 | 9,35 | 7,13 | 305 |
| | | | g. | 75,0 | 8,8 | 8,9 | 7,3 | (19,7) |
| 5 | 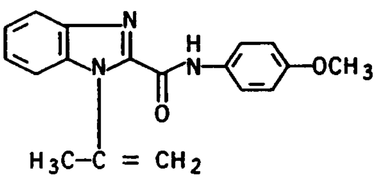 | 113 | b. | 71,0 | 5,92 | 13,08 | 9,97 | 306 |
| | | | g. | 70,9 | 6,0 | 13,2 | 9,9 | (20,8) |
| 6 | 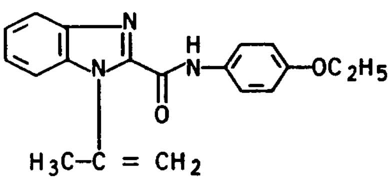 | 107 - 108 | b. | 71,64 | 6,27 | 12,54 | 9,55 | 307 |
| | | | g. | 71,6 | 6,5 | 12,5 | 9,6 | (20,9) |
| 7 | 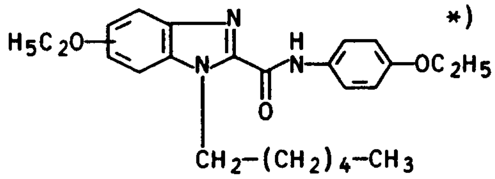 | *) 87 | b. | 70,41 | 7,59 | 10,27 | 11,73 | 323 |
| | | | g. | 70,3 | 7,6 | 10,2 | 11,7 | (21,7) |
| 8 | 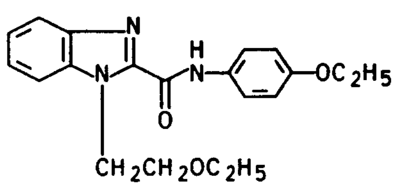 | 88 - 89 | b. | 68,0 | 6,52 | 11,9 | 13,6 | 302 |
| | | | g. | 68,1 | 6,5 | 11,7 | 13,4 | (20,2) |

Tabelle 1 (Fortsetzung)

| Beispiel | Verbindung (I) | Schmp. [°C] | | Analyse C | H | N | O | UV ($CH_3OH$) $\lambda_{max}$ [nm] ($\varepsilon \cdot 10^{-3}$) |
|---|---|---|---|---|---|---|---|---|
| 9 | 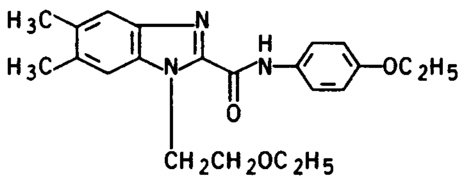 | 139 | b.<br>g. | 69,29<br>69,4 | 7,09<br>6,8 | 11,02<br>11,0 | 12,6<br>12,5 | 315<br>(23,1) |
| 10 | 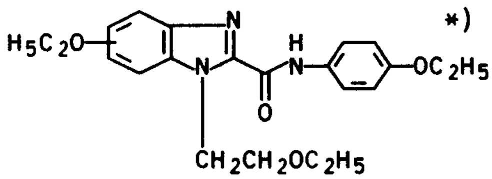 *) | 113 | b.<br>g. | 66,50<br>66,4 | 6,81<br>6,9 | 10,57<br>10,5 | 16,12<br>16,0 | 321<br>(22,3) |

*) Isomerengemische der 5- und 6-Ethoxybenzimidazolderivate

Beispiel 11

28,1 g (0,1 mol) 4'-Ethoxy-benzimidazol-2-carbonsäureanilid wurden zusammen mit 14,1 g 1,5-Dichlorpentan und 20,7 g $K_2CO_3$ in 60 ml Dimethylformamid suspendiert und 11,5 Stunden auf 100 bis 105 °C erhitzt. Die Reaktionsmischung wurde heiß abfiltriert und mit Dimethylformamid nachgewaschen. Nach Abziehen des Lösungsmittels im Vakuum wurde der Rückstand in Methanol heiß gelöst. In der Kälte kristallisierte ein farbloser Feststoff aus, der abgesaugt und mit wenig Methanol gewaschen wurde. Ausbeute: 5,1 g Produkt der Formel

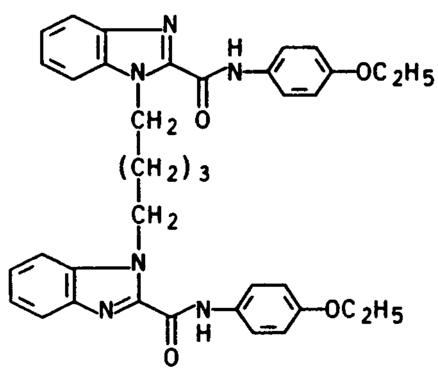

Schmp.: 163 - 165 °C.

| Analyse: $C_{37}$ $H_{38}$ $N_6$ $O_4$ (630,7) | | | | | | | | |
|---|---|---|---|---|---|---|---|---|
| Ber.: | C | 70,47 | H | 6,08 | N | 13,33 | O | 10,15 |
| Gef.: | C | 70,1 | H | 6,1 | N | 13,1 | O | 10,2 |

UV: $\lambda_{max}$ [nm] ($\epsilon \cdot 10^{-3}$) = 305 (36,6)

Beispiel 12 bis 15

Nach der in Beispiel 1 beschriebenen Verfahrensweise wurden durch Umsetzung der entsprechenden Benzimidazol-2-carbonsäureanilide der Formel (II) mit 2-Bromethanol bzw. 4-Chlorbutanol die folgenden in Tabelle 2 beschriebenen hydroxyfunktionalisierten Verbindungen der Formel (V) synthetisiert.

Tabelle 2

| Bsp.-Nr. | Verbindung (I) bzw. (V) | Schmp. [°C] | Analyse | C | H | N | O | UV ($CH_3OH$) $\lambda_{max}$ [nm] ($\varepsilon \cdot 10^{-3}$) |
|---|---|---|---|---|---|---|---|---|
| 12 | 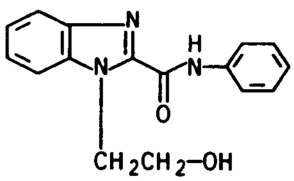 | 177 - 178 | b. | 68,33 | 5,34 | 14,95 | 11,38 | 303 |
| | | | g. | 68,1 | 5,5 | 14,6 | 11,4 | (21,1) |
| 13 | 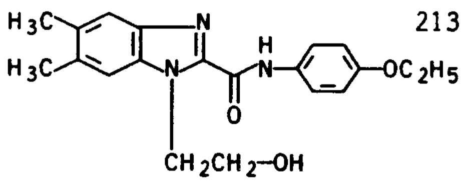 | 213 - 214 | b. | 69,9 | 6,15 | 13,59 | 10,36 | 315 |
| | | | g. | 69,8 | 6,6 | 13,5 | 10,5 | (23,6) |
| 14 | 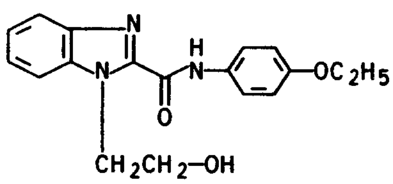 | 146 - 147 | b. | 66,46 | 5,85 | 12,92 | 14,77 | 306 |
| | | | g. | 66,3 | 5,9 | 13,0 | 14,8 | (21,0) |
| 15 | 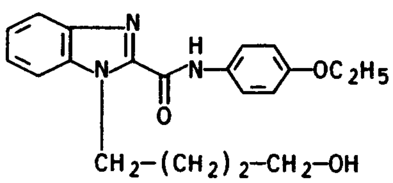 | 123 - 125 | b. | 68,0 | 6,52 | 11,90 | 13,60 | 307 |
| | | | g. | 68,0 | 6,7 | 11,8 | 13,4 | (22,2) |

**Beispiel 16**

Beispiel 16

Zu einer Lösung von 16,3 g (0,05 mol) der Verbindung aus Beispiel 14 in 60 ml trockenen Pyridin wurden bei einer Temperatur zwischen 20 und 35 °C 7,2 g Pivalinsäurechlorid zugetropft. Anschließend wurde 2 h bei 50 °C gerührt, die Reaktionslösung auf 1 l Eiswasser gegossen, 100 ml konzentrierte Salzsäure zugegeben und 0,5 Stunden nachgerührt. Der ausgefallene Niederschlag wurde abgesaugt und mit Eiswasser, Methanol und Ligroin gewaschen. Ausbeute: 18,0 g eines farblosen Feststoffes der Formel

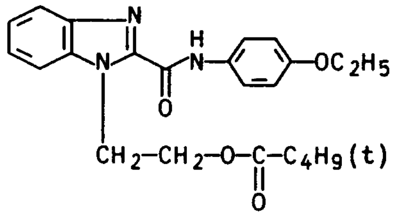

Schmp.: 139 - 140 °C.

| Analyse: $C_{23}$ $H_{27}$ $N_3$ O4 (409,5) | | | | | | | | |
|---|---|---|---|---|---|---|---|---|
| Ber.:<br>Gef.: | C<br>C | 67,48<br>67,5 | H<br>H | 6,61<br>6,8 | N<br>N | 10,27<br>10,2 | O<br>O | 15,64<br>15,3 |

UV: $\lambda_{max}$ [nm] ($\epsilon \cdot 10^{-3}$) = 308 (20,4)

Beispiel 17

Nach der in Beispiel 16 beschriebenen Verfahrensweise wurden 16,3 g der Verbindung aus Beispiel 14 mit 8,5 g p-Toluylsäurechlorid umgesetzt. Nach dem Umkristallisieren aus Ethanol wurden 17,6 g farblose Kristalle der Verbindung mit der Formel

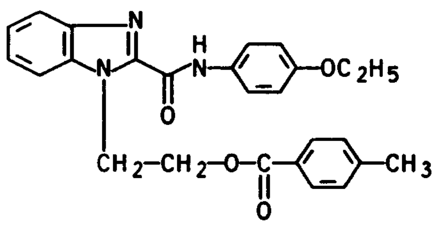

von Schmp.: 150 °C erhalten.

| Analyse: $C_{26}$ $H_{25}$ $N_3$ $O_4$ (443,5) | | | | | | | | |
|---|---|---|---|---|---|---|---|---|
| Ber.:<br>Gef.: | C<br>C | 70,42<br>70,8 | H<br>H | 5,65<br>5,8 | N<br>N | 9,48<br>9,1 | O<br>O | 14,44<br>14,3 |

UV: $\lambda_{max}$ [nm] ($\epsilon \cdot 10^{-3}$) = 302 (19,4)

Beispiel 18

Analog Beispiel 17 wurden 19,5 g der Verbindung aus Beispiel 14 mit 5,6 g Adipinsäuredichlorid umgesetzt. Die Reaktionszeit beträgt 4,5 Stunden. Nach dem Aufarbeiten und Umkristallisieren aus Ethanol wurden 17,8 g eines weißen Feststoffes mit der Formel

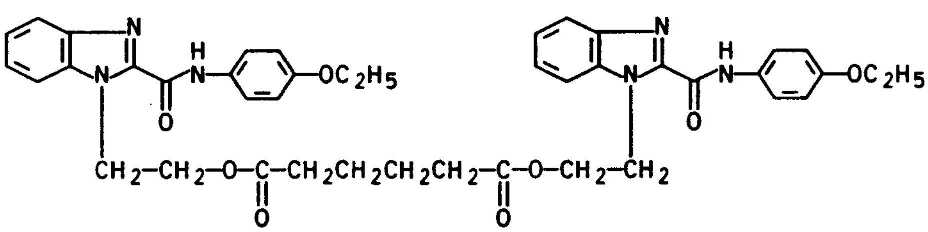

und dem Schmp.: 145 - 147 °C isoliert.

| Analyse: $C_{42}H_{44}N_6O_8$ (760,8) | | | | | | | | |
|---|---|---|---|---|---|---|---|---|
| Ber.: | C | 66,31 | H | 5,8 | N | 11,5 | O | 16,84 |
| Gef.: | C | 66,4 | H | 5,9 | N | 10,9 | O | 16,7 |

UV: $\lambda_{max}$ [nm] ($\epsilon \cdot 10^{-3}$) = 312 (40,5)

Beispiel 19

16,3 g 1-Hydroxyethyl-4′-ethoxy-benzimidazol-2-carbonsäureanilid (Beispiel 14) wurden in 150 ml trockenem Pyridin gelöst. Bei 0 bis 5 °C werden 6,5 g Methacrylsäurechlorid zugetropft und 6,5 Stunden bei Raumtemperatur gerührt. Die Reaktionsmischung wurde auf 800 ml Eiswasser und 100 ml konzentrierte Salzsäure gegossen, der ausgefallene Niederschlag abgesaugt und mit Wasser gewaschen. Das erhaltene Rohprodukt wurde in Aceton heiß gelöst und mit Aktivkohle und Bleicherde behandelt. Nach dem Filtrieren wurde das Lösungsmittel im Vakuum bei 60 - 70 °C abgezogen, wobei 12,8 g eines wachsartigen schwach gelben Produktes der Formel

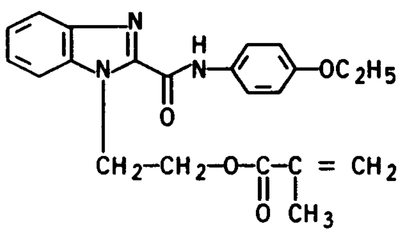

verblieben.

| Analyse: $C_{22}H_{23}N_3O_4$ (393,4) | | | | | | | | |
|---|---|---|---|---|---|---|---|---|
| Ber.: | C | 67,16 | H | 5,89 | N | 10,68 | O | 16,27 |
| Gef.: | C | 66,6 | H | 5,9 | N | 10,3 | O | 16,9 |

UV: $\lambda_{max}$ [nm] ($\epsilon \cdot 10^{-3}$) = 305 (18,5)

Beispiel 20

Nach der in Beispiel 16 beschriebenen Verfahrensweise wurden 16,3 g 1-Hydroxyethyl-4′-ethoxy-benzimidazol-2-carbonsäureanilid (Beispiel 14) mit 15,4 g Palmitinsäurechlorid umgesetzt. Das Rohprodukt wurde in 500 ml Aceton heiß gelöst und mit Aktivkohle und Bleicherde gereinigt. In der Kalte kristallisierten 26,0 g farbloses Produkt der Formel

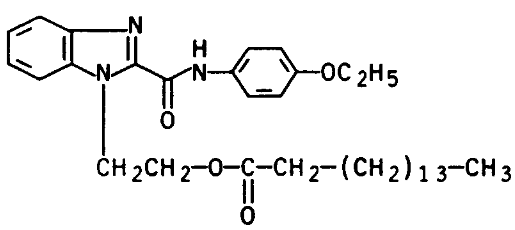

Schmp.: 73 - 76 °C.

| Analyse: $C_{34}$ $H_{49}$ $N_3$ $O_4$ (563,8) | | | | | | | | |
|---|---|---|---|---|---|---|---|---|
| Ber.:<br>Gef.: | C<br>C, | 72,43<br>72,9 | H<br>H | 8,80<br>9,2 | N<br>N | 7,45<br>6,7 | O<br>O | 11,35<br>11,5 |

UV: $\lambda_{max}$ [nm] ($\epsilon \cdot 10^{-3}$) = 305 (18,0)

Beispiel 21

Analog zu Beispiel 20 erhält man durch Verwendung von Ölsäurechlorid 25,5 g eines hellgelben öligen Produktes der Formel

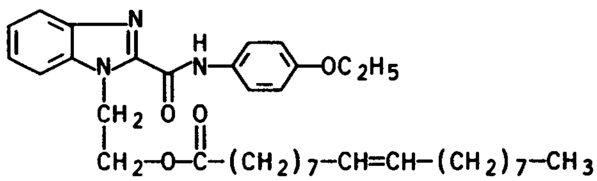

das mit der Zeit wachsartig erstarrt.

| Analyse: $C_{36}$ $H_{51}$ $N_3$ $O_4$ (589,8) | | | | | | | | |
|---|---|---|---|---|---|---|---|---|
| Ber.:<br>Gef.: | C<br>C | 73,31<br>73,5 | H<br>H | 8,72<br>8,7 | N<br>N | 7,12<br>6,6 | O<br>O | 10,85<br>11,1 |

UV: $\lambda_{max}$ [nm] ($\epsilon \cdot 10^{-3}$) = 307 (21,1)

Beispiel 22

Zu einer Lösung von 19,5 g der Verbindung aus Beispiel 14 und 6,1 g Triethylamin in 70 ml Dimethylformamid werden bei Raumtemperatur 7,0 g n-Butylisocyanat zugetropft. Anschließend wird 4 Stunden bei 60 bis 65 °C gerührt. Das Lösungsmittel wird in Vakuum abdestilliert und der Rückstand aus Ethanol umkristallisiert. Es werden 19,9 g farbloses Produkt der Formel

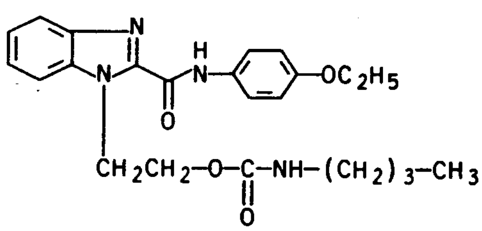

mit dem Schmp. 123-124 °C erhalten.

| Analyse: $C_{23}$ $H_{28}$ $N_4$ $O_4$ (424,5) | | | | | | | | |
|---|---|---|---|---|---|---|---|---|
| Ber.:<br>Gef.: | C<br>C | 65,10<br>65,3 | H<br>H | 6,61<br>6,8 | N<br>N | 13,21<br>13,2 | O<br>O | 15,10<br>15,2 |

UV: $\lambda_{max}$ [nm] ($\epsilon \cdot 10^{-3}$) = 308 (21,3)

Beispiel 23

Analog Beispiel 21 erhält man durch Umsetze mit Phenylisocyanat anstelle von n-Butylisocyanat das Urethan der Formel

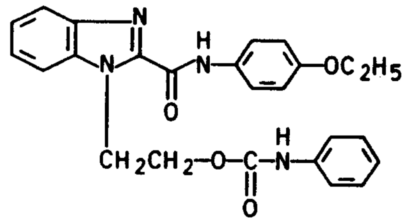

Schmp. 148 - 150 °C.

| Analyse: $C_{25}$ $H_{24}$ $N_4$ $O_4$ (444,5) | | | | | | | | |
|---|---|---|---|---|---|---|---|---|
| Ber.: | C | 67,57 | H | 5,41 | N | 12,61 | O | 14,14 |
| Gef.: | C | 67,5 | H | 5,6 | N | 12,6 | O | 14,4 |

UV: $\lambda_{max}$ [nm] ($\epsilon \cdot 10^{-3}$) = 307 (21,1)

Beispiel 24

19,5 g 1-Hydroxyethyl-4'-ethoxybenzimidazol-2-carbonsäureanilid werden zusammen mit 6,1 g Triethylamin in 100 ml Dimethylformamid gelöst, und bei Raumtemperatur werden 5,1 g Hexamethylendiisocyanat zugetropft. Die Reaktionsmischung wird 5,5 Stunden bei 75 bis 80 °C gerührt, auf 40 °C abkühlen lassen und dann in 350 ml Methanol gegeben. Der ausgefallene Niederschlag wird abgesaugt und mit Methanol und wenig Aceton gewaschen. Ausbeute: 21,1 g Produkt mit der Formel

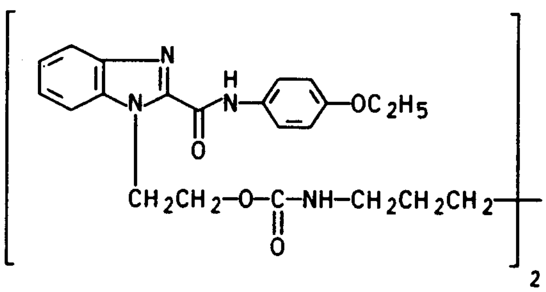

Schmp.: 191 - 193 °C

| Analyse: $C_{44}$ $H_{50}$ $N_8$ $O_8$ (818,8) | | | | | | | | |
|---|---|---|---|---|---|---|---|---|
| Ber.: | C | 64,55 | H | 6,12 | N | 13,69 | O | 15,64 |
| Gef.: | C | 64,4 | H | 6,3 | N | 13,7 | O | 15,8 |

Beispiel 25

Eine Suspension von 28,1 g 4'-Ethoxy-benzimidazol-2-carbonsäureanilid, 14,3 g $K_2CO_3$ und 14,3 g n-Butylglycidylether in 200 ml Dimethylformamid wird 20 Stunden auf 100 - 105 °C unter Rühren erhitzt. Die Reaktionsmischung wird abfiltriert und das Filtrat im Vakuum eingeengt. Der verbleibende ölige Rückstand wird in 450 ml Ethanol heiß gelöst, mit Aktivkohle gereinigt und filtriert. In der Kälte kristallisieren 14,2 g eines farblosen Produktes der Formel

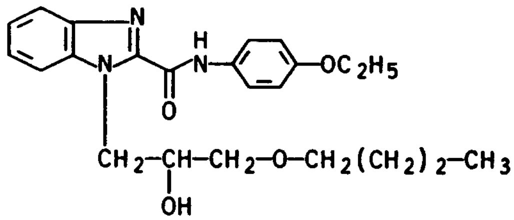

Schmp.: 101 - 102 °C.

| Analyse: $C_{23}$ $H_{29}$ $N_3$ $O_4$ (411,5) | | | | | | | | |
|---|---|---|---|---|---|---|---|---|
| Ber.: | C | 67,13 | H | 7,14 | N | 10,21 | O | 15,52 |
| Gef.: | C | 67,3 | H | 7,1 | N | 10,2 | O | 15,6 |

Beispiel 26

28,1 g 4'-Ethoxy-benzimidazol-2-carbonsäureanilid werden zusammen mit 13,8 g $K_2CO_3$ und 14,6 g Allylglycidylether 5 h auf 100 -105 °C erhitzt. Das Reaktionsgemisch wird heiß abfiltriert. Das Filtrat wird mit Aktivkohle versetzt, 15 Minuten bei 80 °C gerührt und filtriert. Nach dem Abdestillieren des Lösungsmittels verbleibt ein harziger Rückstand, der aus 600 ml Ethanol umkristallisiert wird. Ausbeute: 23,1 g eines farblosen Feststoffes der Formel

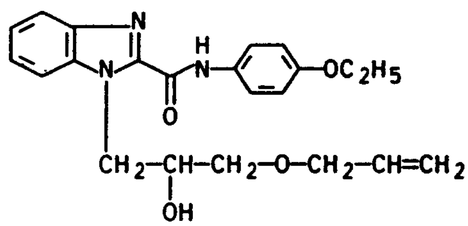

Schmp.: 124 - 126 °C.

| Analyse: $C_{22}$ $H_{25}$ $N_3$ $O_4$ (395,4) | | | | | | | | |
|---|---|---|---|---|---|---|---|---|
| Ber.: | C | 66,84 | H | 6,33 | N | 10,63 | O | 16,20 |
| Gef.: | C | 66,6 | H | 6,5 | N | 10,7 | O | 16,3 |

Beispiel 27

Analog zu Beispiel 26 werden mit n-Dodecylglycidylether 24,5 g farblose Kristalle (Schmp. 79-80 °C) der Formel

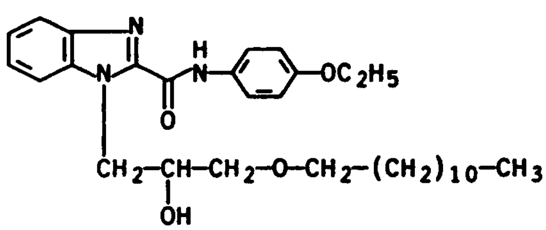

erhalten.

| Analyse: $C_{31}$ $H4_{5}$ $N_3$ $O_4$ (523,6) | | | | | | | | |
|---|---|---|---|---|---|---|---|---|
| Ber.: | C | 71,09 | H | 8,66 | N | 8,02 | O | 12,23 |
| Gef.: | C | 70,6 | H | 8,5 | N | 7,8 | O | 12,2 |

Beispiel 28

Nach der in Beispiel 2 beschriebenen Verfahrensweise wird aus 4′-Ethoxy-benzimidazol-2-carbonsäureanilid und einem 1:1-Gemisch aus 1-Dodecyl- und 1-Tetradecylchlorid ein 1:1-Produktgemisch der Formel

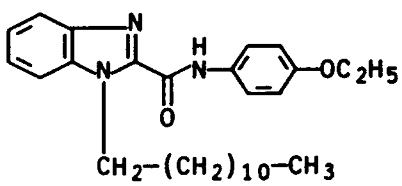

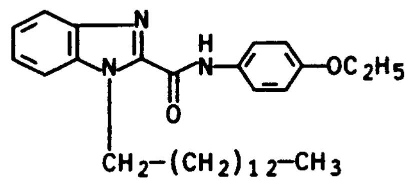

in Form farbloser Kristalle von Schmp. 59-60 °C erhalten.

| Analyse: $C_{28}H_{29}N_3O_2$ / $C_{30}H_{43}N_3O_2$ | | | | | | | | |
|---|---|---|---|---|---|---|---|---|
| Ber.: | C | 75,12 | H | 8,91 | N | 9,06 | O | 6,90 |
| Gef.: | C | 74,9 | H | 9,0 | N | 9,2 | O | 6,9 |

UV: $\lambda_{max}$ [nm] ($\epsilon \cdot 10^{-3}$) = 312 (22,6)

Beispiel 29

84,3 g (0,3 mol) 4′-Ethoxy-benzimidazol-2-carbonsäureanilid werden zusammen mit 58 g (0,42 mol) $K_2CO_3$ und 63,6 g (0,36 mol) Isodecylchlorid in 300 ml Dimethylformamid 4,5 Stunden bei 120 bis 125 °C gerührt. Das Reaktionsgemisch wird heiß abfiltriert, der Rückstand mit 100 ml Dimethylformamid nachgewaschen und die vereinigten Filtrate im Vakuum eingeengt. Der ölige Rückstand wird in 300 ml Essigsäureethylester aufgenommen und mit Aktivkohle und Bleicherde gereinigt. Nach dem Abdestillieren des Lösungsmittels im Vakuum verbleiben 122 g eines hellgelben Öles des Produktes mit der Formel

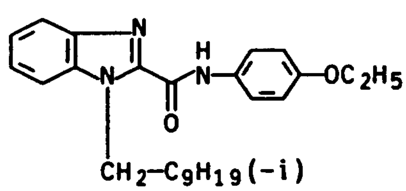

| Analyse: $C_{26}H_{35}N_3O_2$ (421,3) | | | | | | | | |
|---|---|---|---|---|---|---|---|---|
| Ber.: | C | 74,05 | H | 8,38 | N | 9,67 | O | 7,60 |
| Gef.: | C | 74,1 | H | 8,6 | N | 9,9 | O | 7,8 |

UV: $\lambda_{max}$ [nm] ($\epsilon \cdot 10^{-3}$) = 311 (23,1)

Beispiel 30

Nach der in Beispiel 29 beschriebenen Verfahrensweise erhält man bei Verwendung von 4′-Ethoxybenzimidazol-2-carbonsäureanilid und 1-Chlor-2,4,6-trioxadecan das Produkt mit der Formel

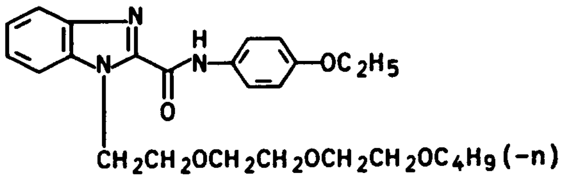

in Form eines hellgelben Öles.
$C_{26}H_{35}N_3O_5$ (469,3)
UV: $\lambda_{max}$ [nm] ($\epsilon \cdot 10^{-3}$) = 312 (21,0)

Beispiel 31

Analog der in Beispiel 29 beschriebenen Verfahrensweise erhält man mit $Cl-CH_2-CH_2\{O-CH_2CH_2\}_2OC_2H_5$ das Produkt mit der Formel

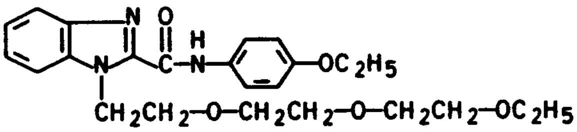

in Form eines gelben Öls.

| $C_{24}H_{31}N_3O_2$ (441,3) | | | | | | | | |
|---|---|---|---|---|---|---|---|---|
| Ber.: | C | 65,35 | H | 7,1 | N | 9,5 | O | 18,1 |
| Gef.: | C | 61,1 | H | 7,1 | N | 9,9 | O | 18,3 |

UV: $\lambda_{max}$ [nm] ($\epsilon \cdot 10^{-3}$) = 312 (22,6)

Beispiel 32

Analog der in Beispiel 29 beschriebenen Verfahrensweise erhält man mit $Cl-CH_2-CH_2\{O-CH_2CH_2\}_2OCH_3$ das Produkt mit der Formel

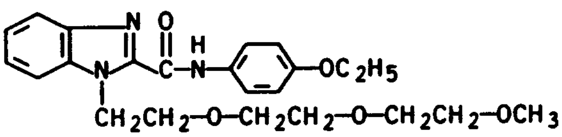

als hellgelbes Öl.

| $C_{23}H_{29}N_3O_5$ (427,5) | | | | | | | | |
|---|---|---|---|---|---|---|---|---|
| Ber.: | C | 64,6 | H | 6,8 | N | 9,8 | O | 18,7 |
| Gef.: | C | 64,1 | H | 6,8 | N | 10,4 | O | 18,7 |

UV: $\lambda_{max}$ [nm] ($\epsilon \cdot 10^{-3}$) = 312 (20,7)

Beispiel 33

Analog der in Beispiel 29 beschriebenen Verfahrensweise erhält man mit 2-n-Hexoxyethylchlorid das Produkt mit der Formel

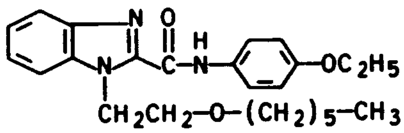

als farblosen Feststoff von Schmp. 66-67 ° C (aus Methanol).

| $C_{24}H_{31}N_3O_3$ (409,5) | | | | | | | | |
|---|---|---|---|---|---|---|---|---|
| Ber.: | C | 70,4 | H | 7,6 | N | 10,3 | O | 11,7 |
| Gef.: | C | 70,3 | H | 7,8 | N | 10,3 | O | 11,9 |

UV: $\lambda_{max}$ [nm] ($\epsilon \cdot 10^{-3}$) = 305 (25,5)

Beispiel 34

Analog der in Beispiel 29 beschriebenen Verfahrensweise erhält man aus 4'-Ethoxybenzimidazol-2-carbonsäureanilid und Iso-nonylchlorid das Produkt mit der Formel

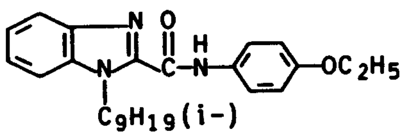

als farblosen Feststoff von Schmp. 120-125 ° C.

| $C_{25}H_{33}N_3O_2$ (407,5) | | | | | | | | |
|---|---|---|---|---|---|---|---|---|
| Ber.: | C | 73,7 | H | 8,2 | N | 10,3 | O | 7,9 |
| Gef.: | C | 73,7 | H | 8,5 | N | 10,4 | O | 8,0 |

UV: $\lambda_{max}$ [nm] ($\epsilon \cdot 10^{-3}$) = 311 (22,8)

Beispiel 35

Analog Beispiel 29 erhält man aus 4'-Ethoxybenzimidazol-2-carbonsäureanilid und Oleylchlorid das Produkt der Formel

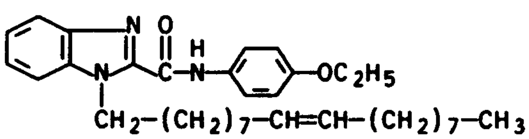

als hellgelbes Öl.

| $C_{34}H_{49}N_3O_2$ (531,4) | | | | | | | | |
|---|---|---|---|---|---|---|---|---|
| Ber.: | C | 76,8 | H | 9,3 | N | 7,9 | O | 6,0 |
| Gef.: | C | 76,3 | H | 9,3 | N | 8,0 | O | 6,2 |

UV: $\lambda_{max}$ [nm] ($\epsilon \cdot 10^{-3}$) = 311 (22,0)

Beispiel 36

Analog Beispiel 29 erhält man aus 4'-Ethoxybenzimidazol-2-carbonsäureanilid und iso-Tridecylchlorid das Produkt der Formel

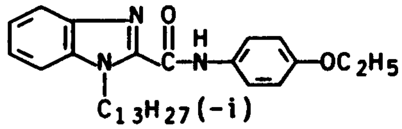

| $C_{29}H_{41}N_3O_2$ (463,6) | | | | | | | | |
|---|---|---|---|---|---|---|---|---|
| Ber.: | C | 75,1 | H | 8,9 | N | 9,1 | O | 6,9 |
| Gef.: | C | 75,3 | H | 9,1 | N | 8,9 | O | 7,2 |

UV: $\lambda_{max}$ [nm] ($\epsilon \cdot 10^{-3}$) = 312 (20,5)

Beispiel 37

Analog der in Beispiel 29 beschriebenen Verfahrensweise erhält man mit $Cl\text{-}CH_2\text{-}CH_2\text{-}(O\text{-}CH_2CH_2)_3OC_2H_5$ das Produkt der Formel

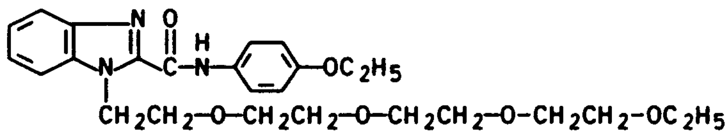

| $C_{26}C_{35}N_3O_6$ (485,3) | | | | | | | | |
|---|---|---|---|---|---|---|---|---|
| Ber.: | C | 64,3 | H | 7,3 | N | 8,7 | O | 19,8 |
| Gef.: | C | 63,4 | H | 7,1 | N | 9,0 | O | 19,7 |

UV: $\lambda_{max}$ [nm] ($\epsilon \cdot 10^{-3}$) = 311 (20,7)

Beispiel 38

Analog der in Beispiel 29 beschriebenen Verfahrensweise erhält man mit $Cl\text{-}CH_2\text{-}CH_2(OCH_2CH_2)_3OCH_3$ das Produkt mit der Formel

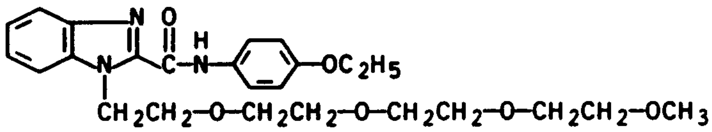

als gellgelbes öl.

| $C_{25}H_{33}N_3O_6$ (471,5) | | | | | | | | |
|---|---|---|---|---|---|---|---|---|
| Ber.: | C | 63,7 | H | 7,1 | N | 8,9 | O | 20,4 |
| Gef.: | C | 63,1 | H | 7,0 | N | 8,8 | O | 20,6 |

UV: $\lambda_{max}$ [nm] ($\epsilon \cdot 10^{-3}$) = 311 (20,4)

B) Anwendungsbeispiele

Anwendungsbeispiel 1 (Lichtstabilisierende Wirkung in Polyurethan)

Herstellung der Belichtungsproben aus Polyurethan
Eine Polyolkomponente der Zusammensetzung

41,9 Teile eines Polyetherols der OH-Zahl von 29,0 das durch Addition von Propylenoxid und Ethylenoxid an Propylenglykol erhalten wurde und ungefähr 84 % primäre Hydroxylgruppen besitzt und 42,5 Teile eine Polyetherols der OH-Zahl von 27,0, das durch Addition von Propylenoxid und Ethylenoxid an Trimethylolpropan erhalten wurde und ungefähr 88 % primäre Hydroxylgruppen besitzt und 8,1 Teile 1,4-Butandiol und 1,724 Teile 25 %ige Lösung von Diazabicyclooctan in 1,4-Butandiol und 0,016 Teile Dibutylzinndilaurat und 0,1 Teile des Siliconstabilisators OS 710 der Firma Bayer und 5,49 Teile Frigen 11 und 0,17 Teile wasser wurde mit den angegebenen Stabilisatoren versetzt und im Mischungsverhältnis 100 : 48,5 mit einem 23,0 %-isocyanatgruppenhaltigen Prepolymeren bei 25 °C Komponenten-und Werkzeugtemperatur zu Prüfplatten verschäumt. Das NCO-Prepolymer wurde hergestellt aus 87,17 Teilen 4,4'-Diphenylmethandiisocyanat und 4,83 Teilen eines Polyetherols mit der OH-Zahl von 250, das durch Addition von Propylenoxid an Propylenglykol erhalten wurde, und 8,0 Teile Dipropylenglykol.

Die Proben wurden im Xenotest®450 belichtet und an den Proben der Yellowness Index (YI) gemäß ASTM D 1925 bestimmt. Die Ergebnisse sind nachfolgend angegeben.

Tabelle 3

| Stabilisator | Konzentration [%] | YI nach ASTM D 1925 | |
|---|---|---|---|
| | | 0 h | 48 h |
| 3.1 Kontrolle | — | 1,4 | 24,1 |
| 3.2 Verbindung aus Beisp. 1 | 1,0 | 1,6 | 12,9 |
| 3.3 Verbindung aus Beisp. 2 | 1,0 | 3,0 | 12,0 |
| 3.4 Verbindung aus Beisp. 9 | 1,0 | 3,4 | 15,0 |
| 3.5 Kombination aus: | | | |
| 2-(2'-Hydroxy-5'-methyl-phenyl)-benztriazol | 0,5 | | |
| + A*) | 0,5 | 2,7 | 14,8 |
| + Triethylenglykol-bis-3--(3-t-butyl-4-hydroxy-5--methylphenyl)-propionat | 0,25 | | |

*) A =

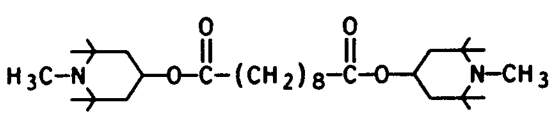

Anwendungsbeispiel 2

Die folgenden in Tabelle 4 aufgeführten Proben wurden analog Anwendungsbeispiel 1 präpariert und geprüft.

Tabelle 4

| Stabilisator | | Konzentration [%] | YI nach ASTM D 1925 | |
|---|---|---|---|---|
| | | | 0 h | 96 h |
| 4.1 | Kontrolle | - | 5,0 | 32,5 |
| 4.2 | Verbindung aus Beisp. 6<br>+ A<br>+ B *) | 0,5<br>0,5<br>0,25 | 4,6 | 21,0 |
| 4.3 | Verbindung aus Beisp. 8<br>+ A<br>+ B | 0,5<br>0,5<br>0,25 | 2,6 | 22,6 |
| 4.4 | Verbindung aus Beisp. 14<br>+ A<br>+ B | 0,5<br>0,5<br>0,25 | 4,7 | 19,1 |
| 4.5 | Verbindung aus Beisp. 22<br>+ A<br>+ B | 0,5<br>0,5<br>0,25 | 4,0 | 18,5 |

B *) = Gemisch aus 1 Gew.-Teil α-Tocopherol und 10 Gew.-Teilen Tris(nonylphenyl)phosphit

Anwendungsbeispiel 3

Flüchtigkeit von Stabilisatoren im Klarlack eines Zweischicht-Metallicsystems

Jeweils 0,4 g Stabilisator werden in 55,6 g spritzfertigem Klarlack, bestehend aus 1500 Teilen Acrylat-Einbrennlack und 167 Teilen Xylol, bis zur vollständigen Lösung gerührt.

Die fertige Lacklösung wird auf ein Quarzglas aufgerakelt (Schichtdicke ca. 40 µm) und 5 Stunden bei 140 °C eingebrannt. Zur Bestimmung des Verlustes an UV-Absorber während des Einbrennvorganges wurden vor und nach dem Einbrennen Absorptionsspektren aufgenommen und aus der Abnahme der optischen Dichte im Absorptionsmaximum der Verlust ermittelt.

Tabelle 5

| Stabilisator | | Prozentualer Verlust nach 5 h bei 140 °C |
|---|---|---|
| 5.1 | 4-Octyloxy-2-hydroxy-benzophenon | 18 |
| 5.2 | 2-(3',5'-Di-t-amyl-2'-hydroxyphenyl)-benztriazol | 83 |
| 5.3 | 2-(3',5'-Di-α,α'-dimethylbenzyl-2'-hydroxyphenyl)-benztriazol | 5 |
| 5.4 | Verbindung aus Bsp. 1 | 5 |
| 5.5 | Verbindung aus Bsp. 2 | 1,7 |
| 5.6 | Verbindung aus Bsp. 4 | 2,8 |
| 5.7 | Verbindung aus Bsp. 8 | 5,7 |
| 5.8 | Verbindung aus Bsp. 9 | 6,3 |
| 5.9 | Verbindung aus Bsp. 10 | 0,5 |
| 5.10 | Verbindung aus Bsp. 11 | 2,4 |
| 5.11 | Verbindung aus Bsp. 14 | 6,5 |
| 5.12 | Verbindung aus Bsp. 17 | 3,3 |

## Patentansprüche

1. Verbindungen der Formel (I)

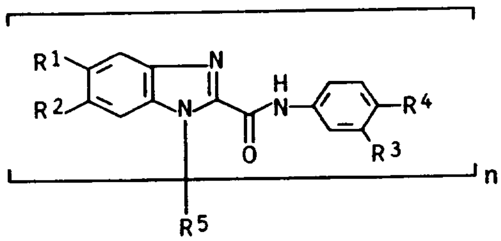

(I)

in der

| | |
|---|---|
| $R^1$ und $R^2$ | unabhängig voneinander für H, Cl, $C_1$- bis $C_4$-Alkyl, $C_1$- bis $C_4$-Alkoxy, gegebenenfalls durch $C_1$- bis $C_4$-Alkyl oder $C_1$- bis $C_4$-Alkoxy substituiertes Phenyl oder $C_7$- bis $C_9$-Phenylalkyl, |
| $R^3$ und $R^4$ | unabhängig voneinander für H, $C_1$- bis $C_{18}$-Alkyl, durch -O-ein- oder mehrfach unterbrochenes $C_3$- bis $C_{18}$-Alkyl, $C_1$- bis $C_{18}$-Alkoxy, durch -O- ein- oder mehrfach unterbrochenes $C_4$- bis $C_{18}$-Alkoxy, gegebenenfalls durch $C_1$- bis $C_4$-Alkyl oder $C_1$- bis $C_4$-Alkoxy substituiertes Phenyl, $C_7$- bis $C_{15}$-Phenylalkyl, Phenoxy, $C_7$- bis $C_{15}$-Phenylalkyloxy, $C_1$- bis $C_{12}$-Alkylcarbonylamino, Benzoylamino, $C_1$- bis $C_{12}$-Alkanoyloxy oder Benzoyloxy, oder |
| $R^3$ und $R^4$ | zusammen für Methylendioxy oder Ethylendioxy, |
| n | für 1 oder 2 und |
| $R^5$ | a) - wenn n = 1 ist - für $C_5$- bis $C_{18}$-Alkyl, $C_2$- bis $C_{10}$-Hydroxyalkyl, $C_5$- bis $C_{12}$-Cycloalkyl, $C_2$-bis $C_{18}$-Alkenyl, wobei Cycloalkyl und Alkenyl durch OH substituiert sein können, durch -O- ein- oder mehrfach unterbrochenes $C_3$-bis $C_{18}$-Alkyl, durch $C_1$- bis $C_4$-Alkyl oder $C_1$- bis $C_4$-Alkoxy substituiertes $C_8$- bis $C_{15}$-Phenylalkyl oder für einen Rest der Formeln |

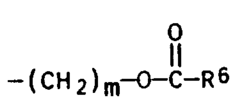 , 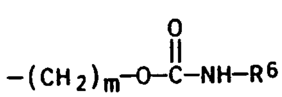 , 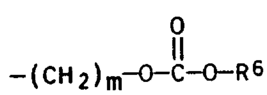 oder

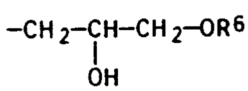

| | |
|---|---|
| | worin |
| m | 2 bis 4 und |
| $R^6$ | $C_1$- bis $C_{18}$-Alkyl, $C_2$- bis $C_{18}$-Alkenyl, $C_5$- bis $C_{12}$-Cycloalkyl, $C_7$- bis $C_{15}$-Phenylalkyl, gegebenenfalls durch $C_1$- bis $C_4$-Alkyl, $C_1$-bis $C_4$-Alkoxy oder Cl substituiertes Phenyl oder Naphthyl bedeuten, oder |
| | b) - wenn n = 2 ist - für $C_2$- bis $C_{10}$-Alkylen, $C_5$- bis $C_{12}$-Cycloalkylen, wobei Alkylen und Cycloalkylen durch -OH substituiert sein können, $C_4$- bis $C_{12}$-Alkenylen, durch -O- ein oder mehrfach unterbrochenes $C_4$- bis $C_8$-Alkylen oder für einen Rest der Formeln |

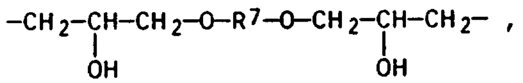 ,

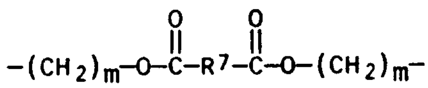 , 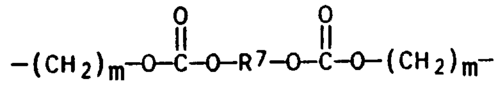

oder 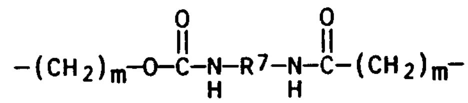 ,

stehen, worin

R7 $C_2$- bis $C_8$-Alkylen, $C_2$- bis $C_8$-Alkenylen, $C_5$- bis $C_{12}$-Cycloalkylen, gegebenenfalls durch $C_1$- bis $C_4$-Alkyl oder $C_1$- bis $C_4$-Alkoxy substituiertes Phenylen, Naphthylen, Diphenylen oder einen Rest der Formeln

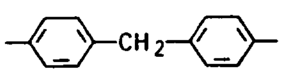 , 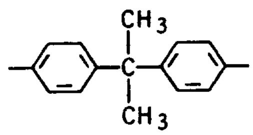 , 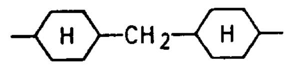 oder

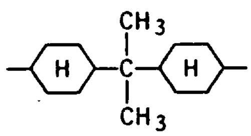

bedeutet.

2. Verbindungen gemäß Anspruch 1, dadurch gekennzeichnet, daß in der Formel

R1 und R2 unabhängig voneinander für H, $C_1$- bis $C_4$-Alkyl, $C_1$- bis $C_4$-Alkoxy oder $C_7$- bis $C_9$-Phenylalkyl,

R3 und R4 unabhängig voneinander für H, $C_1$- bis $C_{12}$-Alkyl, $C_1$- bis $C_{12}$-Alkoxy, durch -O- einfach oder mehrfach unterbrochenes $C_3$- bis $C_{10}$-Alkyl oder $C_4$- bis $C_{12}$-Alkoxy, $C_7$- bis $C_9$-Phenylalkyl, $C_7$- bis $C_9$-Phenylalkoxy, Phenoxy, $C_1$- bis $C_{12}$-Alkylcarbonylamino, Benzoylamino, $C_1$-$C_{12}$-Alkanoyloxy oder Benzoyloxy,

n für 1 und

R5 für $C_5$- bis $C_{18}$-Alkyl, Hydroxy-$C_2$- bis $C_{10}$-alkyl, $C_5$- bis $C_{12}$-Cycloalkyl, $C_2$- bis $C_{18}$-Alkenyl, durch -O- ein- oder mehrfach unterbrochenes $C_3$- bis $C_{18}$-Alkyl, durch $C_1$- bis $C_4$-Alkyl oder $C_1$- bis $C_4$-Alkoxy substituiertes $C_8$- bis $C_{15}$-Phenylalkyl oder für einen Rest der Formeln

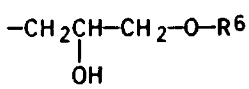 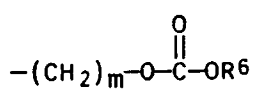 ,

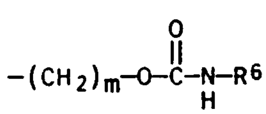 oder 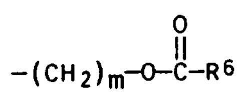

stehen, worin

m 2, 3 oder 4 und

R6 $C_1$- bis $C_{18}$-Alkyl, $C_2$- bis $C_{18}$-Alkenyl, $C_5$- bis $C_{12}$-Cycloalkyl, gegebenenfalls durch $C_1$- bis $C_4$-Alkyl oder $C_1$- bis $C_4$-Alkoxy substituiertes $C_7$- bis $C_{15}$-Phenylalkyl, gegebenenfalls durch $C_1$- bis $C_4$-Alkyl, $C_1$- bis $C_4$-Alkoxy substituiertes Phenyl oder

Naphthyl bedeuten.

**3.** Verbindungen gemäß Anspruch 1, dadurch gekennzeichnet, daß in der Formel

$R^1$ und $R^2$ unabhängig voneinander für H, $C_1$- bis $C_4$-Alkyl, $C_1$- bis $C_4$-Alkoxy oder $C_7$- bis $C_9$-Phenylalkyl,

$R^3$ und $R^4$ unabhängig voneinander für H, $C_1$- bis $C_{12}$-Alkyl, $C_1$- bis $C_{12}$-Alkoxy, durch -O- ein- oder mehrfach unterbrochenes $C_4$- bis $C_{12}$-Alkoxy, $C_7$- bis $C_9$-Phenylalkyl, $C_7$- bis $C_9$-Phenylalkoxy, Phenoxy, $C_1$- bis $C_{12}$-Alkylcarbonylamino, Benzoylamino, $C_1$- bis $C_{12}$-Alkanoyloxy oder Benzoyloxy,

n für 2 und

$R^5$ für $C_2$- bis $C_{10}$-Alkylen oder Hydroxyalkylen, gegebenenfalls durch -OH substituiertes $C_5$- bis $C_{12}$-Cycloalkylen, $C_4$- bis $C_{12}$-Alkenylen, durch -O- ein- oder mehrfach unterbrochenes $C_4$-bis $C_8$-Alkylen, oder für einen Rest der Formeln

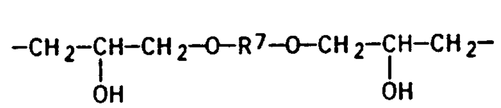
,
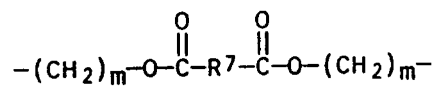

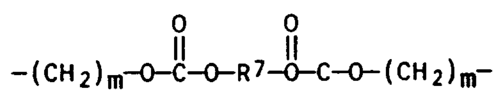
oder

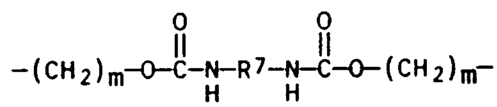

stehen, worin

m 2, 3 oder 4 und

$R^7$ $C_2$- bis $C_8$-Alkylen, $C_2$- bis $C_8$-Alkenylen, $C_5$- bis $C_{12}$-Cycloalkylen, gegebenenfalls durch $C_1$- bis $C_4$-Alkyl oder $C_1$- bis $C_4$-Alkoxy substituiertes Phenylen, Naphthylen, Biphenylen,

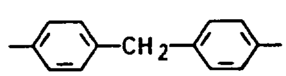
,
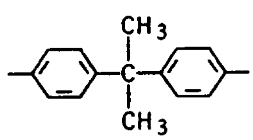
,
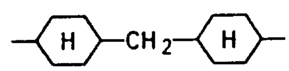

oder
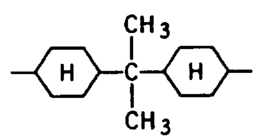

bedeuten.

**4.** Verbindungen gemäß Anspruch 1 oder 2, dadurch gekennzeichnet, daß in der Formel (I) $R^1$ und $R^2$ unabhängig voneinander für H, $C_1$- bis $C_4$-Alkyl, Methoxy, Ethoxy, Benzyl, Phenylethyl, $\alpha$-Methylbenzyl oder $\alpha,\alpha'$-Dimethylbenzyl,

$R^3$ für H,

$R^4$ für $C_1$- bis $C_{12}$-Alkyl, $C_1$- bis $C_{12}$-Alkoxy, Phenoxy, $C_7$- bis $C_9$-Phenylalkoxy, $C_1$- bis $C_{12}$-Alkylcarbonylamino, Benzoylamino, $C_1$- bis $C_{12}$-Alkanoyloxy oder Benzoyloxy,

n für 1 und

$R^5$ für $C_5$- bis $C_{18}$-Alkyl, $C_2$- bis $C_8$-Hydroxyalkyl, $C_2$- bis $C_{18}$-Alkenyl, durch -O- ein- oder

mehrfach unterbrochenes $C_3$- bis $C_{10}$-Alkyl, durch Methyl, Ethyl oder Methoxy substituiertes $C_8$- bis $C_9$-Phenylalkyl oder für einen Rest der Formeln

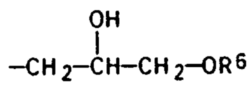, 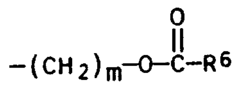 oder 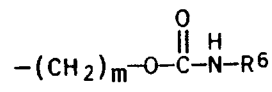

stehen, worin

m 2, 3 oder 4 und

$R^6$ $C_1$- bis $C_{18}$-Alkyl, $C_2$- bis $C_{18}$-Alkenyl, $C_7$- bis $C_9$-Phenylalkyl, gegebenenfalls durch $C_1$- bis $C_4$-Alkyl oder $C_1$- bis $C_4$-Alkoxy substituiertes Phenyl oder Naphthyl bedeuten.

**5.** Verbindungen gemäß Anspruch 1 oder 3, dadurch gekennzeichnet, daß in der Formel

$R^1$ und $R^2$ unabhängig voneinander für H, $C_1$- bis $C_4$-Alkyl, Methoxy, Ethoxy, Benzyl, $\alpha$-Methylbenzyl oder $\alpha,\alpha$-Dimethylbenzyl,

$R^3$ für H,

$R^4$ für $C_1$- bis $C_{12}$-Alkyl, $C_1$- bis $C_{12}$-Alkoxy, Phenoxy, $C_7$- bis $C_9$-Phenylalkoxy, $C_1$- bis $C_{12}$-Alkylcarbonylamino, Benzoylamino, $C_1$-bis $C_{12}$-Alkanoyloxy oder Benzoyloxy,

n für 2 und

$R^5$ für $C_2$- bis $C_{10}$-Alkylen, $C_4$- bis $C_{12}$-Alkenylen, durch -O- ein oder mehrfach unterbrochenes $C_4$- bis $C_8$-Alkylen oder für einen zweiwertigen Rest der Formeln

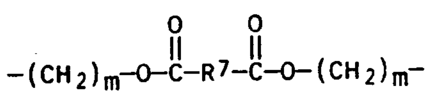 oder 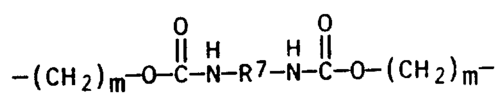

stehen, worin

m 2, 3, 4 und

$R^7$ $C_2$- bis $C_8$-Alkylen, $C_2$- bis $C_8$-Alkenylen, 1,3- oder 1,4-Phenylen, oder Diphenylen bedeuten.

**6.** Verwendung der Verbindungen gemäß den Ansprüchen 1 bis 5 als Lichtschutzmittel für organische Materialien.

**7.** Stabilisiertes organisches Material enthaltend in der Masse 0,1 bis 5 Gew.-%, bezogen auf das organische Material, eine oder mehrere Verbindungen gemäß den Ansprüchen 1 bis 5.

**8.** Stabilisiertes Material nach Anspruch 7, dadurch gekennzeichnet, daß das Material ein synthetisches Polymer ist.

**9.** Stabilisiertes Material gemäß Anspruch 8, dadurch gekennzeichnet, daß das Polymer ein Polyester, Polyamid, heißvernetzbares Acrylharz, thermoplastisches Acrylharz, MF- oder UF-Harz, ABS oder ein Polyurethan ist.

**10.** Lack, enthaltend stabilisiertes Material gemäß Anspruch 9.

## Claims

**1.** Compounds of the formula (I)

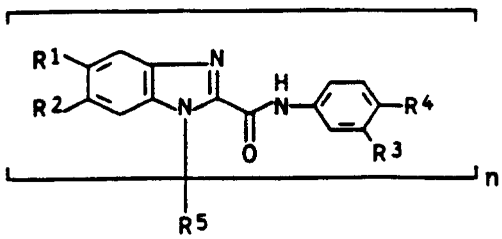

(I)

where

- $R^1$ and $R^2$ are each independently of the other H, Cl, $C_1$-$C_4$-alkyl, $C_1$-$C_4$-alkoxy, unsubstituted or $C_1$-$C_4$-alkyl- or $C_1$-$C_4$-alkoxy-substituted phenyl or $C_7$-$C_9$-phenylalkyl,
- $R^3$ and $R^4$ are each independently of the other H, $C_1$-$C_{18}$-alkyl, $C_3$-$C_{18}$-alkyl which is interrupted one or more times by -O-, $C_1$-$C_{18}$-alkoxy, $C_4$-$C_{18}$-alkoxy which is interrupted one or more times by -O-, unsubstituted or $C_1$-$C_4$-alkyl- or $C_1$-$C_4$-alkoxy-substituted phenyl, $C_7$-$C_{15}$-phenylalkyl, phenoxy, $C_7$-$C_{15}$-phenylalkyloxy, $C_1$-$C_{12}$-alkylcarbonylamino, benzoylamino, $C_1$-$C_{12}$-alkanoyloxy or benzoyloxy, or
- $R^3$ and $R^4$ together are methylenedioxy or ethylenedioxy,
- n is 1 or 2 and
- $R^5$ is a) - when n is 1 - $C_5$-$C_{18}$-alkyl, $C_2$-$C_{10}$-hydroxyalkyl, $C_5$-$C_{12}$-cycloalkyl, $C_2$-$C_{18}$-alkenyl, which cycloalkyl and alkenyl may be substituted by OH, $C_3$-$C_{18}$-alkyl which is interrupted one or more times by -O-, $C_1$-$C_4$-alkyl- or $C_1$-$C_4$-alkoxy-substituted $C_8$-$C_{15}$-phenylalkyl or a radical of the formula

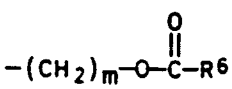 , 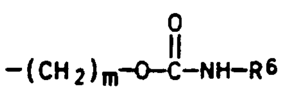 , 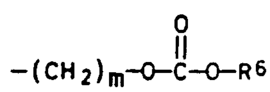 or

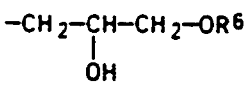

where

- m is from 2 to 4 and
- $R^6$ is $C_1$-$C_{18}$-alkyl, $C_2$-$C_{18}$-alkenyl, $C_5$-$C_{12}$-cycloalkyl, $C_7$-$C_{15}$-phenylalkyl, unsubstituted or $C_1$-$C_4$-alkyl-, $C_1$-$C_4$-alkoxy or Cl-substituted phenyl or naphthyl, or b) - when n is 2 - $C_2$-$C_{10}$-alkylene, $C_5$-$C_{12}$-cycloalkylene, which alkylene and cycloalkylene may be substituted by -OH, $C_4$-$C_{12}$-alkenylene, $C_4$-$C_8$-alkylene which is interrupted one or more times by -O-, or a radical of the formula

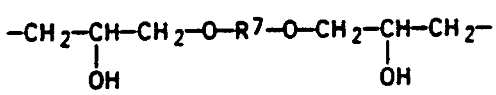 ,

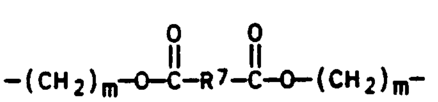 , 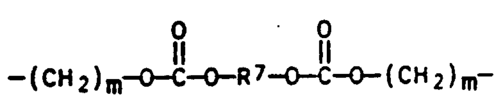

or 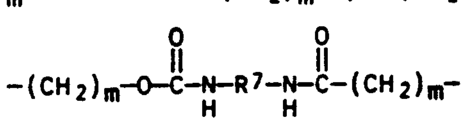 ,

where

$R^7$ is $C_2$-$C_8$-alkylene, $C_2$-$C_8$-alkenylene, $C_5$-$C_{12}$-cycloalkylene, unsubstituted or $C_1$-$C_4$-alkyl- or $C_1$-$C_4$-alkyl- or $C_1$-$C_4$-alkoxy-substituted phenylene, naphthylene, diphenylene or a radical of the formula

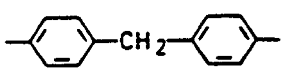 , 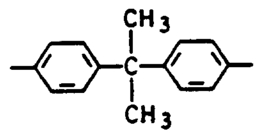 , 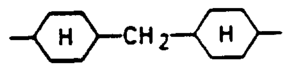 or

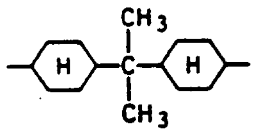

**2.** Compounds as claimed in claim 1, wherein in the formula

$R^1$ and $R^2$ are each independently of the other H, $C_1$-$C_4$-alkyl, $C_1$-$C_4$-alkoxy or $C_7$-$C_9$-phenylalkyl,

$R^3$ and $R^4$ are each independently of the other H, $C_1$-$C_{12}$-alkyl, $C_1$-$C_{12}$-alkoxy, $C_3$-$C_{10}$-alkyl which is interrupted one or more times by -O-, $C_4$-$C_{12}$-alkoxy which is interrupted one or more times by -O-, $C_7$-$C_9$-phenylalkyl, $C_7$-$C_9$-phenylalkoxy, phenoxy, $C_1$-$C_{12}$-alkylcarbonylamino, benzoylamino, $C_1$-$C_{12}$-alkanoyloxy or benzoyloxy,

n is 1 and

$R^5$ is $C_5$-$C_{18}$-alkyl, $C_2$-$C_{10}$-hydroxyalkyl, $C_5$-$C_{12}$-cycloalkyl, $C_2$-$C_{18}$-alkenyl, $C_3$-$C_{18}$-alkyl which is interrupted one or more times by -O-, $C_1$-$C_4$-alkyl- or $C_1$-$C_4$-alkoxy-substituted $C_8$-$C_{15}$-phenylalkyl or a radical of the formula

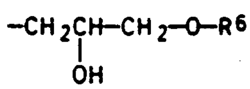 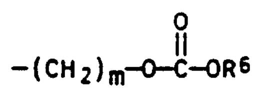 ,

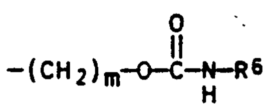 or 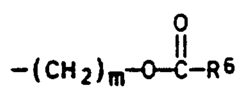

where

m is 2, 3 or 4 and

$R^6$ is $C_1$-$C_{18}$-alkyl, $C_2$-$C_{18}$-alkenyl, $C_5$-$C_{12}$-cycloalkyl, unsubstituted or $C_1$-$C_4$-alkyl- or $C_1$-$C_4$-alkoxy-substituted $C_7$-$C_{15}$-phenylalkyl, unsubstituted or $C_1$-$C_4$-alkyl- or $C_1$-$C_4$-alkoxy-substituted phenyl or naphthyl.

**3.** Compounds as claimed in claim 1, wherein in the formula

$R^1$ and $R^2$ are each independently the other H, $C_1$-$C_4$-alkyl, $C_1$-$C_4$-alkoxy or $C_7$-$C_9$-phenylalkyl,

$R^3$ and $R^4$ are each independently of the other H, $C_1$-$C_{12}$-alkyl, $C_1$-$C_{12}$-alkoxy, $C_4$-$C_{12}$-alkoxy which is interrupted one or more times by -O-, $C_7$-$C_9$-phenylalkyl, $C_7$-$C_9$-phenylalkoxy, phenoxy, $C_1$-$C_{12}$-alkylcarbonylamino, benzoylamino, $C_1$-$C_{12}$-alkanoyloxy or benzoyloxy,

n is 2 and

$R^5$ is $C_2$-$C_{10}$-alkylene or hydroxyalkylene, unsubstituted or -OH-substituted $C_5$-$C_{12}$-cycloalkylene, $C_4$-$C_{12}$-alkenylene, $C_4$-$C_8$-alkylene which is interrupted one or more times by -O-, or a radical of the formula

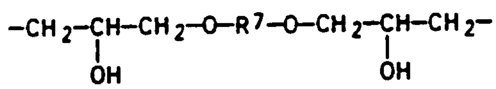, 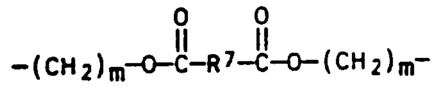

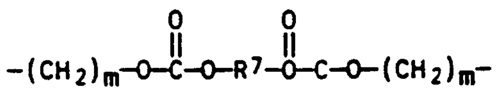 or

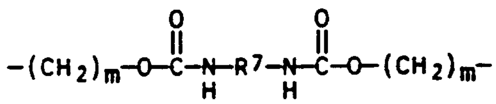

m is 2, 3 or 4 and

$R^7$ is $C_2$-$C_8$-alkylene, $C_2$-$C_8$-alkenylene, $C_5$-$C_{12}$-cycloalkylene, unsubstituted or $C_1$-$C_4$-alkyl- or $C_1$-$C_4$-alkoxy-substituted phenylene, naphthylene, diphenylene,

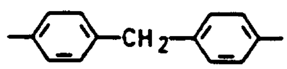, 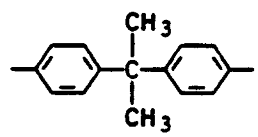, 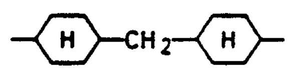

or 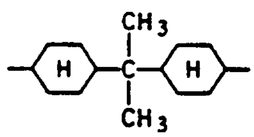

**4.** Compounds as claimed in claim 1 or 2, wherein in the formula (I) $R^1$ and $R^2$ are each independently of the other H, $C_1$-$C_4$-alkyl, methoxy, ethoxy, benzyl, phenylethyl, $\alpha$-methylbenzyl or $\alpha,\alpha'$-dimethylbenzyl,

$R^3$ is H,

$R^4$ is $C_1$-$C_{12}$-alkyl, $C_1$-$C_{12}$-alkoxy, phenoxy, $C_7$-$C_9$-phenylalkoxy, $C_1$-$C_{12}$-alkylcarbonylamino, benzoylamino, $C_1$-$C_{12}$-alkanoyloxy or benzoyloxy,

n is 1 and

$R^5$ is $C_5$-$C_{18}$-alkyl, $C_2$-$C_8$-hydroxyalkyl, $C_2$-$C_{18}$-alkenyl, $C_3$-$C_{10}$-alkyl which is interrupted one or more times by -O-, methyl-, ethyl- or methoxy-substituted $C_8$ or $C_9$-phenylalkyl or a radical of the formula

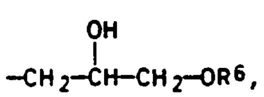, 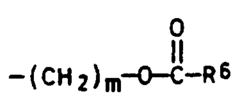 or 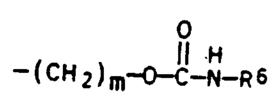

where

m is 2, 3 or 4 and

$R^6$ is $C_1$-$C_{18}$-alkyl, $C_2$-$C_{18}$-alkenyl, $C_7$-$C_9$-phenylalkyl, unsubstituted or $C_1$-$C_4$-alkyl- or $C_1$-$C_4$-alkoxy-substituted phenyl or $C_1$-$C_4$-alkyl- or $C_1$-$C_4$-alkoxy-substituted naphthyl.

**5.** Compounds as claimed in claim 1 or 3, wherein in the formula

$R^1$ and $R^2$ are each independently of the other H, $C_1$-$C_4$-alkyl, methoxy, ethoxy, benzyl, $\alpha$-methylbenzyl or $\alpha,\alpha$-dimethylbenzyl,

$R^3$ is H,

$R^4$ is $C_1$-$C_{12}$-alkyl, $C_1$-$C_{12}$-alkoxy, phenoxy, $C_7$-$C_9$-phenylalkoxy, $C_1$-$C_{12}$-alkylcarbonylamino, benzoylamino, $C_1$-$C_{12}$-alkanoyloxy or benzoyloxy,

n is 2 and

$R^5$ is $C_2$-$C_{10}$-alkylene, $C_4$-$C_{12}$-alkenylene, $C_4$-$C_8$-alkylene which is interrupted one or

more times by -0-, or a divalent radical of the formula

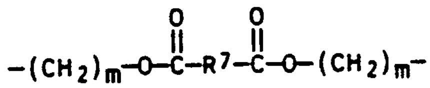

or 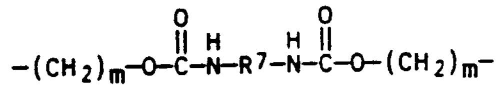

where

m is 2, 3 or 4 and

$R^7$ is $C_2$-$C_8$-alkylene, $C_2$-$C_8$-alkenylene, 1,3- or 1,4-phenylene or diphenylene.

**6.** Use of the compounds of claim 1 to 5 as light stabilizers for organic materials.

**7.** A stabilized organic material containing in the mass from 0.1 to 5 % by weight, based on the organic material, of one or more compounds as claimed in claims 1 to 5.

**8.** A stabilized material as claimed in claim 7, wherein the material is a synthetic polymer.

**9.** A stabilized material as claimed in claim 8, wherein the polymer is a polyester, a polyamide, a hot-crosslinkable acrylic resin, a thermoplastic acrylic resin, an MF or UF resin, ABS or a polyurethane.

**10.** A surface coating containing a stabilized material as claimed in claim 9.

## Revendications

**1.** Composés de la formule (I)

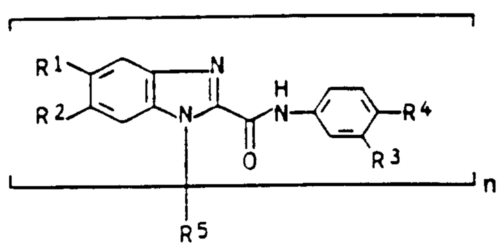

(I)

dans laquelle

$R^1$ et $R^2$ représentent, chacun indépendamment l'un de l'autre, un atome d'hydrogène, un atome de chlore, un radical alkyle en $C_1$-$C_4$, alcoxy en $C_1$-$C_4$, phényle éventuellement substitué par des radicaux alkyle en $C_1$-$C_4$ ou alcoxy en $C_1$-$C_4$, ou un radical phényl-($C_7$-$C_9$)alkyle,

$R^3$ et $R^4$ représentent, chacun indépendamment l'un de l'autre, un atome d'hydrogène, un radical alkyle en $C_1$-$C_{18}$, alkyle en $C_3$-$C_{18}$ une ou plusieurs fois interrompus par -O-, alcoxy en $C_1$-$C_{18}$, alcoxy en $C_4$-$C_{18}$ une ou plusieurs fois interrompus par -O-, phényle éventuellement substitué par des radicaux alkyle en $C_1$-$C_4$ ou alcoxy en $C_1$-$C_4$, phényl-($C_7$-$_{15}$)alkyle, phénoxy, phényl($C_7$-$C_{15}$)alkyloxy, alkyl($C_1$-$C_{12}$)carbonylamino, benzoylamino, alcanoyloxy en $C_1$-$C_{12}$ ou benzoyloxy, ou bien

$R^3$ et $R^4$ forment ensemble le radical méthylènedioxy ou éthylènedioxy,

n est égal à 1 ou à 2 et

$R^5$ représente a) - lorsque n est égal à 1 - un radical alkyle en $C_5$-$C_{18}$, hydroxyalkyle en $C_2$-$C_{10}$, cycloalkyle en $C_5$-$C_{12}$, alcényle en $C_2$-$C_{18}$, où les radicaux cycloalkyle et alcényle peuvent être substitués par des groupes OH, alkyle en $C_3$-$C_{18}$ une ou plusieurs fois interrompus par -O-, phényl($C_8$-$C_{15}$)alkyle substitué par des radicaux alkyle en $C_1$-$C_4$ ou alcoxy en $C_1$-$C_4$, ou représente un groupe de la formule

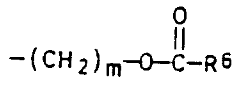 , 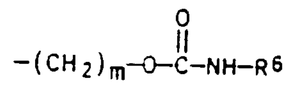 , 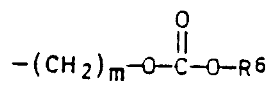 ou

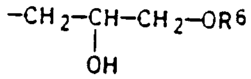

dans laquelle

m a une valeur qui varie de 2 à 4 et

$R^6$ représente un radical alkyle en $C_1$-$C_{18}$, alcényle en $C_2$-$C_{18}$, cycloalkyle en $C_5$-$C_{12}$-, phényl($C_7$-$C_{15}$)alkyle, naphtyle ou phényle éventuellement substitué par des radicaux alkyle en $C_1$-$C_4$, alcoxy en $C_1$-$C_4$ ou des atomes de chlore, ou bien

b) - lorsque n est égal à 2, - représente un radical alkylène en $C_2$-$C_{10}$, cyloalkylène en $C_5$-$C_{12}$, où les radicaux alkylène et cycloalkylène peuvent être substitués par des radicaux OH, alcénylène en $C_4$-$C_{12}$,alkylène en $C_4$-$C_8$ une ou plusieurs fois interrompus par -O-, ou un radical de la formule

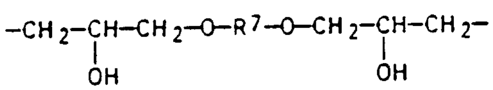 ,

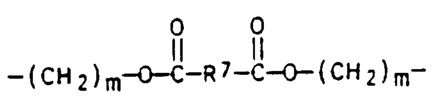 , 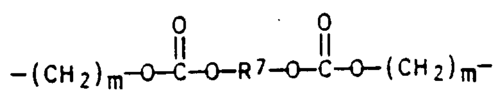

ou 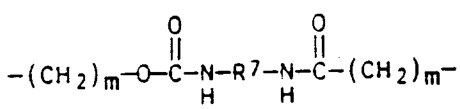 ,

dans laquelle

$R^7$ représente un radical alkylène en $C_2$-$C_8$, alcénylène en $C_2$-$C_8$, cycloalkylène en $C_5$-$C_{12}$, diphénylène, naphtylène, phénylène éventuellement substitué par des radicaux alkyle en $C_1$-$C_4$ ou alcoxy en $C_1$-$C_4$ ou un radical de la formule

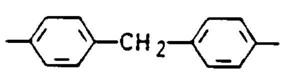 , 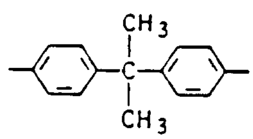 , 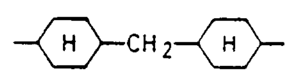 ou

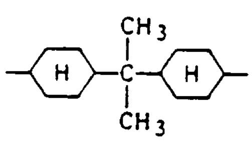 .

**2.** Composés suivant la revendication 1, caractérisés en ce que, dans la formule,

$R^1$ et $R^2$ représentent, chacun indépendamment l'un de l'autre, un atome d'hydrogène, un radical alkyle en $C_1$-$C_4$, alcoxy en $C_1$-$C_4$, ou phényl($C_7$-$C_9$)alkyle,

$R^3$ et $R^4$ représentent, chacun indépendamment l'un de l'autre, un atome d'hydrogène, un radical alkyle en $C_1$-$C_{12}$, alcoxy en $C_1$-$C_{12}$, alkyle en $C_3$-$C_{10}$ une ou plusieurs fois interrompus par -O-, alcoxy en $C_4$-$C_{12}$, phényl($C_7$-$C_9$)alkyle, phényl($C_7$-$C_9$)alcoxy, phénoxy, alkyl($C_1$-$C_{12}$)carbonylamino, benzoylamino, alcanoyloxy en $C_1$-$C_{12}$, ou benzoyloxy,

n est égal à 1 et

$R^5$ représente un radical alkyle en $C_5$-$C_{18}$, hydroxyalkyle en $C_2$-$C_{10}$, cycloalkyle en $C_5$-$C_{12}$, alcényle en $C_2$-$C_{18}$, alkyle en $C_3$-$C_{18}$ une ou plusieurs fois interrompus par -O-, phényl($C_8$-$C_{15}$)alkyle substitué par des radicaux alkyle en $C_1$-$C_4$ ou alcoxy en $C_1$-$C_4$,

ou un radical de la formule

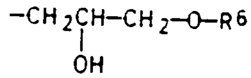

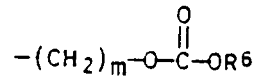 ,

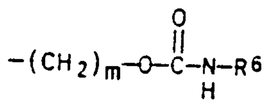 ou 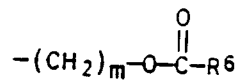

dans laquelle

m est égal à 2, 3 ou 4 et

$R^6$ représente un radical alkyle en $C_1$-$C_{18}$, alcényle en $C_2$-$C_{18}$, cycloalkyle en $C_5$-$C_{12}$, phényl-($C_7$-$C_{15}$)alkyle éventuellement substitué par des radicaux alkyle en $C_1$-$C_4$ ou alcoxy en $C_1$-$C_4$, naphtyle ou phényle éventuellement substitué par des radicaux alkyle en $C_1$-$C_4$, alcoxy en $C_1$-$C_4$.

**3.** Composés suivant la revendication 1, caractérisés en ce que, dans la formule,

$R^1$ et $R^2$ représentent, chacun indépendamment l'un de l'autre, un atome d'hydrogène, un radical alkyle en $C_1$-$C_4$, alcoxy en $C_1$-$C_4$, ou phényl($C_7$-$C_9$)alkyle,

$R^3$ et $R^4$ représentent, chacun indépendamment l'un de l'autre, un atome d'hydrogène, un radical alkyle en $C_1$-$C_{12}$, alcoxy en $C_1$-$C_{12}$, alcoxy en $C_4$-$C_{12}$ une ou plusieurs fois substitués par -O-, phényl($C_7$-$C_9$)alkyle, phényl($C_7$-$C_9$)alcoxy, phénoxy, alkyl($C_1$-$C_{12}$)-carbonylamino, benzoylamino, alcanoyloxy en $C_1$-$C_{12}$, ou benzoyloxy,

n est égal à 2 et

$R^5$ représente un radical hydroxyalkylène ou alkylène en $C_2$-$C_{10}$, cycloalkylène en $C_5$-$C_{12}$ éventuellement substitué par des radicaux -OH, alcénylène en $C_4$-$C_{12}$,alkylène en $C_4$-$C_8$ une ou plusieurs fois interrompus par -O-, ou un radical de la formule

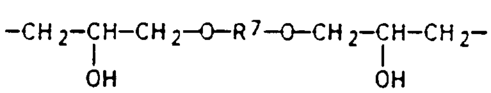 , 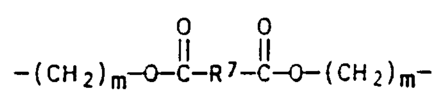

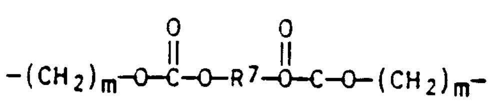

ou 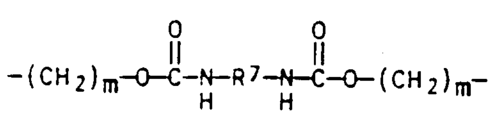

dans laquelle

m est égal à 2, 3 ou 4 et

$R^7$ représente un radical alkylène en $C_2$-$C_8$, alcénylène en $C_2$-$C_8$, cycloalkylène en $C_5$-$C_{12}$, biphénylène, naphtylène, phénylène éventuellement substitué par des radicaux alkyle en $C_1$-$C_4$ ou alcoxy en $C_1$-$C_4$,

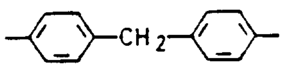, 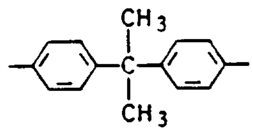, 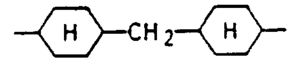

ou 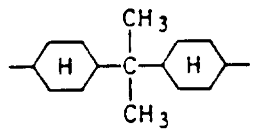.

4. Composés suivant la revendication 1 ou 2, caractérisés en ce que, dans la formule (I), $R^1$ et $R^2$ représentent, chacun indépendamment l'un de l'autre, un atome d'hydrogène, un radical alkyle en $C_1$-$C_4$, méthoxy, éthoxy, benzyle, phényléthyle, $\alpha$-méthylbenzyle ou $\alpha,\alpha'$-diméthylbenzyle,
   - $R^3$ représente un atome d'hydrogène,
   - $R^4$ représente un radical alkyle en $C_1$-$C_{12}$, alcoxy en $C_1$-$C_{12}$, phénoxy, phényl($C_7$-$C_9$)alcoxy, alkyl($C_1$-$C_{12}$)carbonylamino, benzoylamino, alcanoyloxy en $C_1$-$C_{12}$, ou benzoyloxy,
   - n est égal à 1 et
   - $R^5$ représente un radical alkyle en $C_5$-$C_{18}$, hydroxyalkyle en $C_2$-$C_8$, alcényle en $C_2$-$C_{18}$, alkyle en $C_3$-$C_{10}$ une ou plusieurs fois interrompus par -O-, phényl($C_8$-$C_9$)alkyle substitué par des radicaux méthyle, éthyle, ou méthoxy, ou un radical de la formule

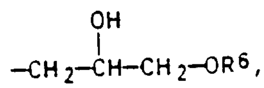, 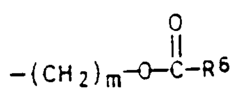 ou 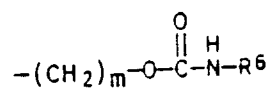

   dans laquelle
   - m est égal à 2, 3 ou 4 et
   - $R^6$ représente un radical alkyle en $C_1$-$C_{18}$, alcényle en $C_2$-$C_{18}$, phényl($C_7$-$C_9$)alkyle, naphtyle, ou phényle éventuellement substitué par des radicaux alkyle en $C_1$-$C_4$ ou alcoxy en $C_1$-$C_4$.

5. Composés suivant la revendication 1 ou 3, caractérisés en ce que, dans le formule,
   - $R^1$ et $R^2$ représentent, chacun indépendamment l'un de l'autre, un atome d'hydrogène, un radical alkyle en $C_1$-$C_4$, méthoxy, éthoxy, benzyle, $\alpha$-méthylbenzyle ou $\alpha,\alpha$-diméthylbenzyle,
   - $R^3$ représente un atome d'hydrogène,
   - $R^4$ représente un radical alkyle en $C_1$-$C_{12}$, alcoxy en $C_1$-$C_{12}$, phénoxy, phényl($C_7$-$C_9$)-alcoxy, alkyl($C_1$-$C_{12}$)carbonylamino, benzoylamino, alcanoyloxy en $C_1$-$C_{12}$, ou benzoyloxy,
   - n est égal à 2 et
   - $R^5$ représente un radical alkylène en $C_2$-$C_{10}$, alcénylène en $C_4$-$C_{12}$, alkylène en $C_4$-$C_8$ une ou plusieurs fois interrompus par -O-, ou un radical bivalent de la formule

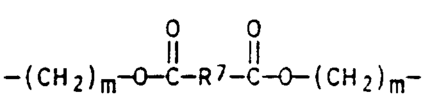 ou 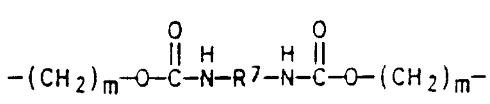

   dans laquelle
   - m est égal à 2, 3 ou 4 et
   - $R^7$ représente un radical alkylène en $C_2$-$C_8$, alcénylène en $C_2$-$C_8$, 1,3- ou 1,4-phénylène, ou diphénylène.

6. Utilisation des composés suivant l'une quelconque des revendications 1 à 5, à titre d'agents photoprotecteurs pour des matières organiques.

**7.** Matière organique stabilisée, qui contient, dans la masse, de 0,1 à 5% en poids, par rapport à la matière organique, d'un ou plusieurs composés suivant l'une quelconque des revendications 1 à 5.

**8.** Matière stabilisée suivant la revendication 7, caractérisée en ce que cette matière est un polymère synthétique.

**9.** Matière stabilisée suivant la revendication 8, caractérisée en ce que le polymère est un polyester, un polyamide, une résine acrylique thermoréticulable, une résine acrylique thermoplastique, une résine MF ou UF, de l'ABS ou un polyuréthanne.

**10.** Laque ou peinture contenant une matière stabilisée suivant la revendication 9.